# EUROPEAN PATENT SPECIFICATION

(11) **EP 4 210 799 B1**
(45) Date of publication and mention of the grant of the patent: **27.05.2026**
(21) Application number: 21867580.9
(22) Date of filing: 09.09.2021
(51) Int. Cl.: A61M 16/06, A61B 1/267, A61M 39/00, A61M 16/01, A61M 39/12, A61M 16/00, A61B 90/00

(54) **VACUUM SHIELD ASSEMBLY FOR ATTACHMENT TO MEDICAL MASKS AND INTUBATION ASSEMBLY TO PROTECT FROM AIRBORNE ILLNESSES**
VAKUUMSCHILDANORDNUNG ZUR BEFESTIGUNG AN MEDIZINISCHEN MASKEN UND INTUBATIONSANORDNUNG ZUM SCHUTZ VOR LUFTERKRANKUNGEN
ENSEMBLE DE PROTECTION SOUS VIDE POUR LA FIXATION À DES MASQUES MÉDICAUX ET ENSEMBLE D'INTUBATION POUR LA PROTECTION CONTRE DES MALADIES AÉROPORTÉES

(30) Priority: 09.09.2020 US 202063075862 P; 09.09.2020 US 202063075890 P; 11.02.2021 US 202117173724; 17.02.2021 US 202117177432; 04.03.2021 US 202117191823
(43) Date of publication of application: 19.07.2023
(73) Proprietor: Safer Medical Products, LLC, Branson, MO 65616 (US)
(72) Inventor: BRADY, Rob, Sarasota, FL 31763 (US); VERGIN, Matt, St. Petersburg, FL 33703 (US); JENNINGS, Barry, Largo, FL 33774 (US); MACFARLANE, Steve, Bradenton, FL 34205-1425 (US); WINTERHALTER, Mike, Nokomis, FL 34275-441 (US); BLUBAUGH, Richard, Branson West, MO 65737 (US); RANDALL, Craig, Branson, MO 65616 (US); DENEVAN, Misty, Branson, MO 65616 (US); BAKER, Todd, Walnut Shade, MO 65711 (US)
(74) Representative: Torner, Juncosa I Associats, SL
(86) International application number: PCT/US2021/049619
(87) International publication number: WO 2022/056099

(56) References cited:
- WO-A1-2021/207292
- CN-A- 105 797 246
- CN-U- 206 007 759
- US-A- 5 370 110
- US-A- 5 676 133
- US-A1- 2002 045 796
- US-A1- 2009 020 128
- US-A1- 2016 074 268
- US-A1- 2016 107 006
- US-A1- 2021 307 872
- US-A1- 2021 346 624
- US-B2- 10 188 814
- US-B2- 7 647 928
- ANONYMOUS: "Product Information Sheet Exhalo Shield", SOVEREIGN MEDICAL, INC., 3 April 2020 (2020-04-03), XP055930207, Retrieved from the Internet <URL:https://sovmed.com/wp-content/uploads/2020/07/EXHALO-Introduction-Summary-Soverign-Medical-ed3.pdf> [retrieved on 20220613]

## Description

### Field of Invention

The present invention relates to attachments to masks for medical procedures, as well as protective shields for intubation, endoscope, bronchoscope, or other procedures that may generate aerosol or respiratory pathogens.

### Background

Medical masks may be used for or for nebulizing a patient or used for Non-Invasive Positive Pressure Ventilation (NIPPV), Bi-level Positive Airway Pressure (BIPAP), Bag-Valve-Mask Resuscitator (BVM), Demand-Valve Resuscitator (DVR), or Constant Positive Airway Pressure (CPAP). Of the masks that currently exist, none are believed to provide a truly efficient means for vacuuming air to create negative pressure, or for implementing a nebulizing or positive pressure procedure and at the same time using negative pressure to vacuum exhaled air from the patient. Accordingly, the industry would benefit by providing a vacuum shield assembly for attachment to a medical mask that may be used for vacuuming exhaled air from patient during nebulization, BIPAP, CPAP, or during delivery of oxygen. Such a vacuum shield assembly would provide the added benefit of at least partially reducing contact to the mask and/or face of the patient, which may help to counter the risk of contagion of airborne illnesses, e.g., influenza, covid-19, etc., providing added protection to medical providers and staff involved in these procedures and in at least partially reducing the development of fomites from exhaled or aerosolized particles or droplets. Additionally, a benefit in the industry would be provided if such a vacuum shield assembly would be disposable as it would further reduce such risk of contagion. An even further benefit would be provided if such a vacuum shield assembly would be sufficiently versatile to be used as a primary and/or a secondary air vacuuming component, and/or a nebulizing component. Yet a further benefit would be realized if this vacuum shield assembly would be provided in different shapes and sizes to correspond to the geometry and size of the underlying face mask.

In addition, the spread of airborne illnesses poses a serious health risk, not only to individuals within the community, but also to health practitioners. This is relevant to patients that have contracted an airborne illness, for example a respiratory illness such as influenza, and more recently COVID-19. Such ill patients may require health practitioners to perform a procedure, for example an intubation, endoscopy, bronchoscopy, or other procedures, especially those requiring fiberoptic devices. During the process of performing the procedure, the health practitioners may be at risk of contagion of the airborne illness. Thus, a benefit would be realized by providing an intubation assembly that can act as a shield to cover the face of an ill patient. It would be ideal if such an intubation assembly would allow a health practitioner to insert a laryngoscope, endoscope, bronchoscope, or other fiberoptic device through the shield. Another benefit would also be realized if the intubation assembly would also allow the health practitioner to insert an endotracheal tube through the shield. An even further benefit would be realized if the shield comprised a substantially transparent material that would permit visibility of the patient and the intubation area. Yet a further benefit would be realized if such an intubation assembly would be provided with various operative components that would be capable of being connected to a vacuum system to provide for a negative pressure that may at least partially removed exhaled air from the patient. Another benefit would be realized if such a shield assembly would be provided as a standalone component that could be used on its own, not tied to a system, to protect other individuals and the environment from exhaled patient particles.

US2016107006-A1 discloses a helmet for anesthesia that is adapted to keep confined anesthetic gases and to administer them in a non-invasive fashion to a patient laying on an operating table, allowing to provide oxygen and/or anesthetic gases through tubes and fast access to the patient's head in case of emergency is described. The helmet has a lower half-shell and an upper half-shell, the lower half-shell being anatomically shaped to receive and support the nape and the neck of the laying patient, the upper half-shell and the lower half-shell having fastening means for fastening one to the other and being configured to be fitted one to the other and to the neck or torso of the patient to form a substantially airtight enclosure for enclosing head of the patient, at least one inlet port for gas supply and at least one outlet port for gas evacuation being on the lower half-shell and/or the upper half-shell.

US5370110-A discloses an anesthesia scavenging hood that is adapted to fit over the head of a surgical patient and to prevent the contamination of operating rooms with waste anesthetic vapor and nitrous oxide gas. The hood enables placement of an endotracheal tube or other breathing or anesthesia circuit into the patient's airway. The hood may be sealed about the endotracheal tube and about the patient. The hood also includes a port having disposed therein a suction tube which communicates waste vapor and gas out of the hood and outside of the operating room with the aid of a vacuum source.

US10188814-B2 discloses a surgical mask for administering and/or scavenging medical gases and that includes a nasal mask and an oral mask that envelops the nasal mask. The nasal mask is secured to the oral mask and may be removable from it.

US7647928-B2 discloses a component for an inhalation device comprising a first flow channel for inhalation, a second flow channel for exhalation, a mouthpiece forming a part of the first flow channel, a filter assigned to the second flow channel and a protection element which circumferentially surrounds the mouthpiece and is sealingly connected the mouthpiece at the end opposing the tip of the mouthpiece.

### Summary

The present invention is defined by the vacuum shield assembly in accordance with claim 1. Additional embodiments are defined in the dependent claims 2-7.

### 1. VACUUM SHIELD ASSEMBLY FOR ATTACHMENT TO MEDICAL MASKS

The present invention is directed to a vacuum shield assembly intended for attachment to an existing mask. As used herein, an "existing mask" refers to a suction mask, a mask configured for attachment to a nebulizer, BIPAP, CPAP, BVM, DVR or another related mask, including a mask configured for the delivery of oxygen to the patient, that is already disposed on the head and/or face of a patient. Accordingly, the vacuum shield assembly of the present invention may serve as a primary and/or a secondary suction or vacuum mechanism, which in some embodiments may be connected to a negative pressure vacuum. The vacuum shield assembly generally comprises a shield body and a retaining assembly. The retaining assembly may be used to connect the vacuum shield to a vacuum tube connected to a negative pressure vacuum. The retaining assembly may also be attached to a nebulizer unit or component thereof, or to the oxygen supply tube of a BIPAP or CPAP mask. Additionally, the shield body may comprise a lower segment. The shield body may be configured with or without a circular access opening in the convexity of the shield body that will allow a BVM, or DVR to connect to an existing mask by way of the access opening in order to facilitate the vacuuming of exhaled air during said procedures. The lower segment may further define an interior or inside of the shield body and may comprise a connecting portion disposed in fluid communication with the retaining assembly and the vacuum tube. The shield body may be configured and dimensioned to correspond to the geometry of the existing mask. As an example, the shield body may comprise a substantially concave configuration and/or a variety of shapes, including, but not limited to, a substantially triangular or substantially ovoidal shape. However, other shapes of the shield body are possible, which may also to correspond to the shape of the existing mask and/or the shape of the face and/or head of the patient. As such, it is within the scope of the present invention that the vacuum shield assembly according to the present invention at least partially remove exhaled infectious particles, for example, from a patient that has a respiratory illness. As a result, it is contemplated that such increased removal of exhaled infectious particles at least partially reduce the risk of contagion of medical practitioners and staff assisting with these types of procedures and/or the contamination of physical objects in the vicinity (fomites).

Further embodiments of the present invention comprise a system configured to remove exhaled air from a patient wearing a medical masks. In such embodiments, it is contemplated that a portable vacuum unit be provided and connected to a vacuum tube that itself connects to the vacuum shield assembly, i.e., to the shield body, to create a negative pressure on an interior of the shield body and remove exhaled air. The innovative system may be provided with a retaining assembly, if it is desirable to connect to a component of an existing medical mask, or without a retaining assembly, in embodiments where it is desirable to dispose the shield body directly on the face of the patient.

### II. INTUBATION ASSEMBLY TO PROTECT FROM AIRBORNE ILLNESSES

The present invention relates to an intubation assembly and shield assembly that may at least partially reduce the risk of contagion of airborne illnesses, including from a patient to healthcare personnel, i.e., physician, nurse, assistant, etc. The present invention also relates to a method of using the inventive intubation assembly. The intubation assembly and shield assembly according to the present invention at least partially reduces exposure of healthcare personnel and others to exhaled infectious particles such as viruses and bacteria. The intubation assembly and shield assembly according to the present invention at least partially allows for protection against exhaled infectious particles. As such, it is contemplated that the inventive intubation assembly may be used as a standalone component, i.e., without being tied to a system that may provide an oxygen supply, air suctioning or nebulization, so that it may protect the environment surrounding the patient from the spread of exhaled contaminated particles. Further, the intubation assembly and shield assembly according to the present invention may at least partially provide a seal between the laryngoscope, endoscope, bronchoscope, or other fiberoptic device and the patient at the location where it is fitted and/or inserted. Furthermore, the intubation assembly and shield assembly according to the present invention may also at least partially provide a seal at the area where the endotracheal tube is inserted or otherwise passed through. Such a configuration of the intubation assembly and shield assembly, including in the geometry of a shield body, may substantially define an operable arrangement in conjunction with negative pressure vacuums. As such, the intubation assembly and shield assembly according to the present invention, when disposed in such operable arrangement, may at least partially remove the patient's exhaled air. This in turn, may protect the healthcare personnel involved in the procedure by at least partially reducing exposure to exhaled infectious particles.

Further embodiments of the present invention comprise a system configured to remove exhaled air from a patient in connection with an intubation or other related procedure. In such embodiments, it is contemplated that a portable vacuum unit be provided and connected to a vacuum tube that itself connects to the shield assembly, i.e., to the shield body, to create a negative pressure on an interior of the shield body and remove exhaled air. The innovative system may be provided with a shield body that may be placed around the face area of the patient to perform an intubation procedure through the mouth of the patient. Alternatively, the innovative system may be provided with a shield body that may be placed around the neck area of the patient to perform an intubation procedure around an opening on the neck area of the patient.

The intubation assembly comprises an intubation apparatus assembly and a shield assembly. The intubation apparatus assembly may be operatively disposed on a shield assembly. The shield assembly is generally connected to a negative pressure vacuum or otherwise vacuum system. The intubation apparatus assembly may comprise an intubation apparatus, including, but not necessarily limited to a laryngoscope, endoscope, bronchoscope, or other fiberoptic apparatus. The intubation apparatus may be placed on a correspondingly dimensioned sleeve of the intubation apparatus assembly. The sleeve should comprise a geometry such that a health practitioner should be able to hold it with a hand(s). The shield assembly comprises a body with a plurality of side segments. The shield assembly may also comprise a first transparent component with a shield opening disposed thereon. The shield opening may be used for insertion of the laryngoscope assembly. By way of example, the first transparent component may comprise a clear silicone sheet with an opening disposed thereon. The shield assembly may also comprise a second transparent component with at least one longitudinally disposed slot for insertion of an endotracheal tube, and in some embodiments, an endoscope, bronchoscope, or other fiberoptic apparatus.

### Brief Description of the Drawings

FIG. 1 is a perspective view of one embodiment of the vacuum shield assembly according to the present invention attached to nebulizer mask.
FIG. 2 is a perspective view of another embodiment of the vacuum shield assembly according to the present invention for use with a nebulizer mask.
FIG. 3 is a perspective view of yet another embodiment of the vacuum shield assembly according to the present invention for use with a nebulizer mask.
FIG. 4 is a perspective and partially exploded view of a further embodiment of the vacuum shield assembly according to the present invention.
FIG. 5 is a perspective view of one embodiment of a retaining assembly of the vacuum shield assembly according to the present invention.
FIG. 6 is a perspective view of one embodiment of the vacuum shield assembly according to the present invention attached to a BIPAP or CPAP mask.
FIG. 7 is a perspective view of another embodiment of the vacuum shield assembly according to the present invention for use with a BIPAP or CPAP mask.
FIG. 8 is a perspective view of yet another embodiment of the vacuum shield assembly according to the present invention for use with a BIPAP or CPAP mask.
FIG. 9 is a perspective and partially exploded view of an even further embodiment of the vacuum shield assembly according to the present invention.
FIG. 10 is a perspective view of yet a further embodiment of the vacuum shield assembly according to the present invention.
FIG. 11 is a perspective view of another embodiment of a retaining assembly of the vacuum shield assembly according to the present invention.
FIG. 12A is a perspective view of one embodiment of a shield body of the vacuum shield assembly according to the present invention comprising a second opening.
FIG. 12B is a perspective view of another embodiment of a shield body of the vacuum shield assembly according to the present invention comprising a second opening.
FIG. 12C is a perspective view of even another embodiment of a shield body of the vacuum shield assembly according to the present invention comprising a second opening.
FIG. 12D is a perspective view of yet another embodiment of a shield body of the vacuum shield assembly according to the present invention comprising a second opening and connected to a BVM or DVR unit.
FIG. 13A is a perspective view of one embodiment of a shield body of the vacuum shield assembly according to the present invention comprising a vacuum attachment disposed on the shield body.
FIG. 13B is a perspective view of another embodiment of a shield body of the vacuum shield assembly according to the present invention comprising a vacuum attachment disposed on the shield body.
FIG. 14A is a perspective view of yet another embodiment of a shield body of the vacuum shield assembly according to the present invention comprising a vacuum attachment disposed on the shield body.
FIG. 14B is a perspective view of an even further embodiment of a shield body of the vacuum shield assembly according to the present invention comprising a vacuum attachment disposed on the shield body.
FIG. 15A is a perspective view of one embodiment of a shield body of the vacuum shield assembly according to the present invention comprising a vacuum attachment disposed on the shield body and connected to a BVM or DVR unit.
FIG. 15B FIG. 15A is a perspective view of another embodiment of a shield body of the vacuum shield assembly according to the present invention comprising a vacuum attachment disposed on the shield body and connected to a BVM or DVR unit.
FIG. 16A is a top view of one embodiment of a vacuum attachment according to the vacuum shield assembly of the present invention.
FIG. 16B is a top view of another embodiment of a vacuum attachment according to the vacuum shield assembly of the present invention.
FIG. 16C is a top view of a further embodiment of a vacuum attachment according to the vacuum shield assembly of the present invention.
FIG. 16D is a top view of yet another embodiment of a vacuum attachment according to the vacuum shield assembly of the present invention.
FIG. 16E is a top view of an even further embodiment of a vacuum attachment according to the vacuum shield assembly of the present invention.
FIG. 16F is a top view of another embodiment of a vacuum attachment according to the vacuum shield assembly of the present invention.
FIG. 16G is a top view of a further embodiment of a vacuum attachment according to the vacuum shield assembly of the present invention.
FIG. 16H is a top view of an even further embodiment of a vacuum attachment according to the vacuum shield assembly of the present invention.
FIG. 16I is a top view of yet another embodiment of a vacuum attachment according to the vacuum shield assembly of the present invention.
FIG. 17 is a perspective view of one embodiment of the system according to the present invention.
FIG. 18A is a perspective view of disassembled components of one embodiment of the system according to the present invention.
FIG. 18B is a perspective view of another embodiment of the system according to the present invention.
FIG. 19A is a perspective side view of one embodiment of the shield body and retaining assembly of the system according to the present invention.
FIG. 19B is a perspective side view of another embodiment of the shield and retaining assembly of the system according to the present invention before attachment to a nebulizer.
FIG. 19C is a perspective side view of yet another embodiment of the shield and retaining assembly of the system according to the present invention attached to a nebulizer.
FIG. 19D is a perspective side view of a further embodiment of the shield and retaining assembly of the system according to the present invention disposed on an existing medical mask attached to a patient.
FIG. 20A is a perspective side view of one embodiment of the shield body and retaining assembly of the system according to the present invention.
FIG. 20B is a perspective side view of another embodiment of the shield and retaining assembly of the system according to the present invention before attachment to an oxygen supply tube.
FIG. 20C is a perspective side view of yet another embodiment of the shield and retaining assembly of the system according to the present invention attached to an oxygen supply tube.
FIG. 20D is a perspective side view of a further embodiment of the shield and retaining assembly of the system according to the present invention disposed on an existing medical mask attached to a patient.
FIG. 21A is a perspective view of a portion of one embodiment of the system according to the present invention comprising a strap for an existing mask and a retaining assembly attached to a nebulizer.
FIG. 21B is a perspective view of a portion of another embodiment of the system according to the present invention comprising a strap for an existing mask and a retaining assembly attached to a nebulizer.
FIG. 21C is a perspective view of a portion of yet another embodiment of the system according to the present invention comprising a strap for an existing mask and a retaining assembly attached to a nebulizer.
FIG. 21D is a perspective view of one embodiment of the system according to the present invention comprising a body shield attached to a medical mask and a patient.
FIG. 22 is a diagrammatic representation of one embodiment of the method according to the present invention for removing exhaled air from a patient.
FIG. 23A is a perspective view of one embodiment of the intubation assembly according to the present invention.
FIG. 23B is a perspective view of another embodiment of the intubation assembly according to the present invention.
FIG. 23C is a perspective view of yet another embodiment of the intubation assembly according to the present invention.
FIG. 23D is a perspective view of one embodiment of the shield assembly according to the present invention.
FIG. 24 is a perspective view of one embodiment of the laryngoscope assembly of the intubation assembly according to the present invention.
FIG. 25 is a perspective view of one embodiment of the shield assembly of the intubation assembly according to the present invention.
FIG. 26 is a perspective view of another embodiment of the shield assembly of the intubation assembly according to the present invention being operated by a user.
FIG. 27 is a side view of one embodiment of the shield assembly of the intubation assembly according to the present invention being operated by a user.
FIG. 28 is a side view of another embodiment of the shield assembly of the intubation assembly according to the present invention being operated by a user.
FIG. 29 is a side view of yet another embodiment of the shield assembly of the intubation assembly according to the present invention being operated by a user.
FIG. 30 is a side view of a patient intubated with the intubation assembly removed.
FIG. 31 is a perspective view of one embodiment of the shield assembly of the intubation assembly according to the present invention comprising offset structures.
FIG. 32 is a front view of one embodiment of the shield assembly of the intubation assembly according to the present invention comprising offset structures and being operatively disposed on the head of a patient.
FIG. 33 is a diagrammatic representation of one embodiment of the method of using the intubation assembly according to the present invention.
FIG. 34 is a perspective view of one embodiment of the system according to the present invention.
FIG. 35 is a perspective view of disassembled components of one embodiment of the system according to the present invention.
FIG. 36A is a perspective side view of one embodiment of the shield body of the system according to the present invention.
FIG. 36B is a perspective side view of one embodiment of the shield body, sleeve and vacuum tube of the system according to the present invention.
FIG. 36C is a perspective side view of another embodiment of the shield body, sleeve and vacuum tube according to the present invention.
FIG. 36D is a perspective side view of yet another embodiment of the shield body, sleeve and vacuum tube according to the present invention.
FIG. 36E is a perspective view of a further embodiment of the shield body, sleeve and vacuum tube according to the present invention.
FIG. 36F is a perspective view of an intubated patient using the system according to the present invention.
FIG. 37A is a top view of one embodiment of shield body of the system according to the present invention.
FIG. 37B is a perspective view of a portion of one embodiment of the system according to the present invention comprising a strap disposed on the shield body.
FIG. 37C is a perspective view of a portion of another embodiment of the system according to the present invention comprising a strap disposed on the shield body.
FIG. 37D is a perspective view of a portion of yet another embodiment of the system according to the present invention comprising a strap disposed on the shield body.
FIG. 37E is a perspective view of a portion of a further embodiment of the system according to the present invention comprising a strap disposed on the shield body.
FIG. 38A is a perspective view of one embodiment of the shield body of the system according to the present invention.
FIG. 38B is a perspective view of another embodiment of the shield body of the system according to the present invention disposed on the head of a patient.

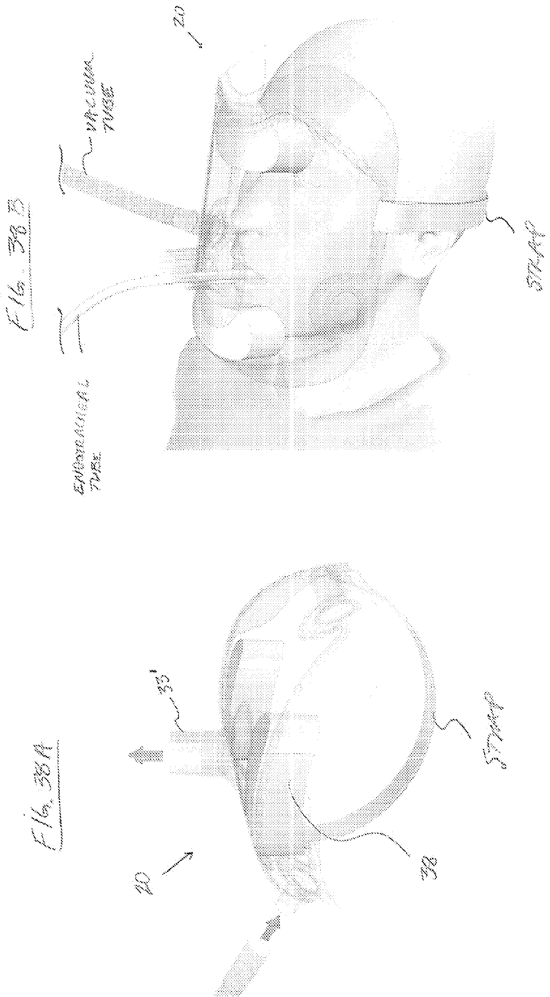

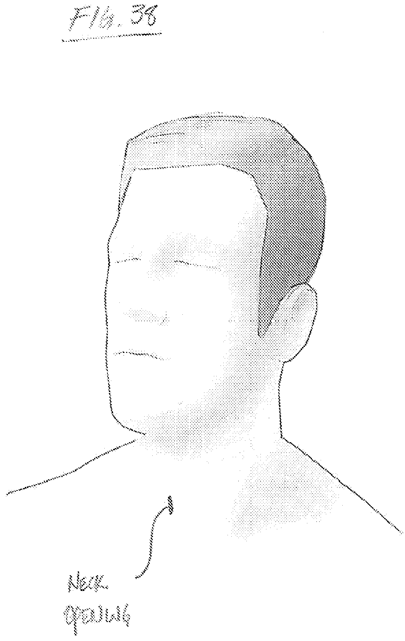

FIG. 38 is a perspective view of a patient with an opening around the neck area.

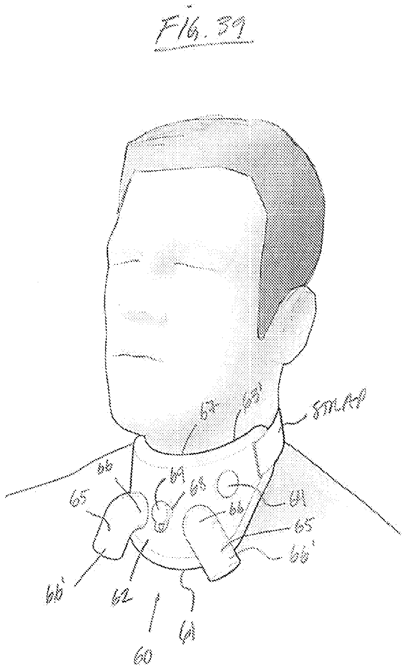

FIG. 39 is a perspective view of one embodiment of the shield body according to the system of the present invention.

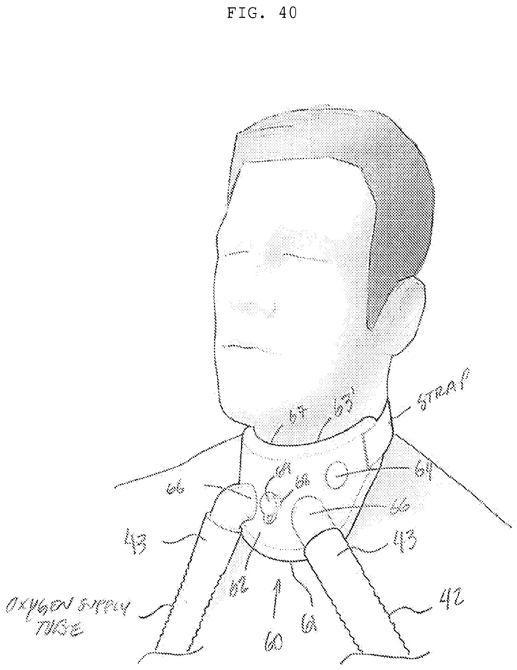

FIG. 40 is a perspective view of another embodiment of the shield body according to the system of the present invention.

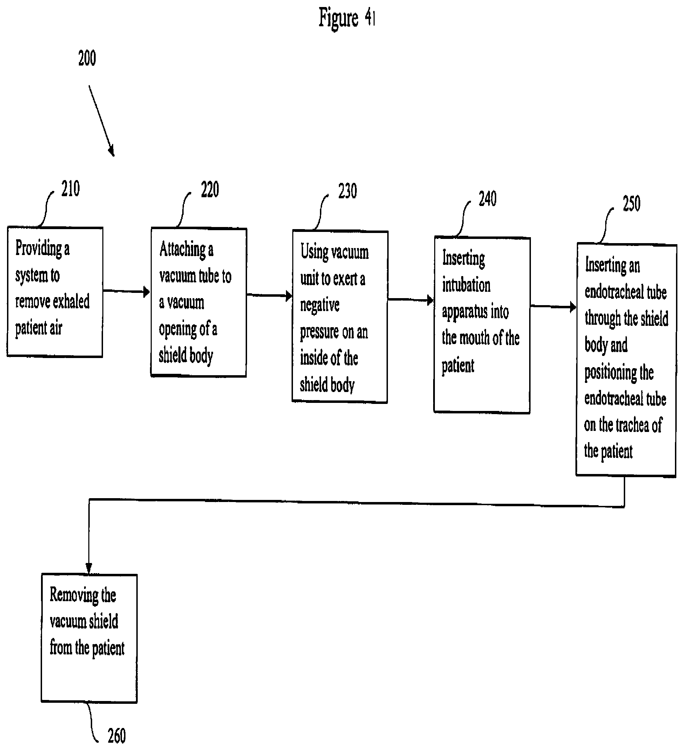

FIG. 41 is a diagrammatic representation of one embodiment of the method according to the present invention for removing exhaled air from a patient.

### Detailed Description

### I. VACUUM SHIELD ASSEMBLY FOR ATTACHMENT TO MEDICAL MASKS

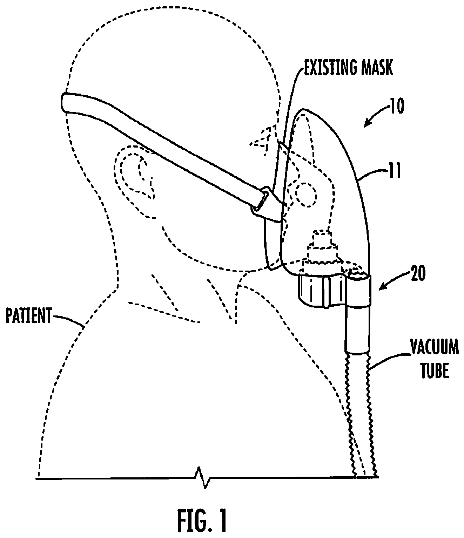

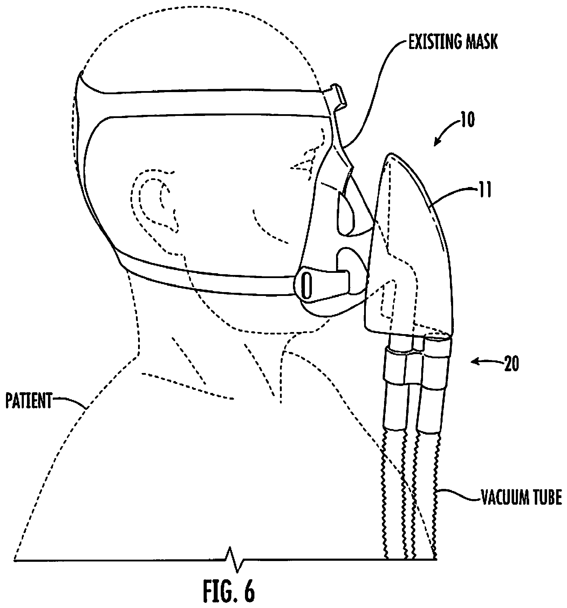

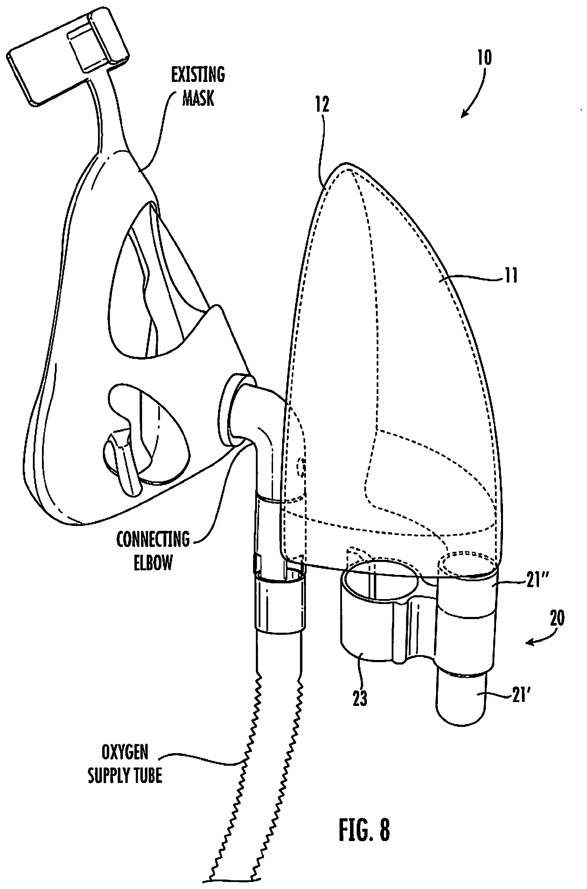

With initial reference to Figures 1-4, 6-10 and 12, the present invention is directed to a vacuum shield assembly 10. The vacuum shield assembly 10 according to the present invention is intended to be disposed on the head and/or face of a patient that is already wearing a medical mask, and is intended to at least partially extract exhaled air from the patient. For example, and as is perhaps best shown in Figures 1 and 6, the vacuum shield assembly 10 may be attached to a mask already disposed on the head and/or face of a patient. The vacuum shield assembly 10 may be connected to a vacuum tube such that it may at least partially extract exhaled air from the patient, including, for example, between the already disposed medical mask and the inside of a shield body 11 of the vacuum shield assembly 10. The vacuum shield assembly 10 may serve as a primary suction or vacuum mechanism, or alternatively, as secondary suction or vacuum mechanism. As an example, and as is shown in Figures 6-8, the vacuum shield assembly 10 may be attached to a BIPAP mask, CPAP mask and/or a mask configured to provide oxygen delivery to a patient, which is already disposed on the head and/or face of a patient. Other possible existing medical masks that could be used in connection with the present invention include a face mask, a face tent, a venture mask, and/or a non-rebreather. As a further example, and as is shown in Figures 1-3, the vacuum shield assembly 10 may be attached to a nebulizing mask already disposed on the head and/or face of a patient.

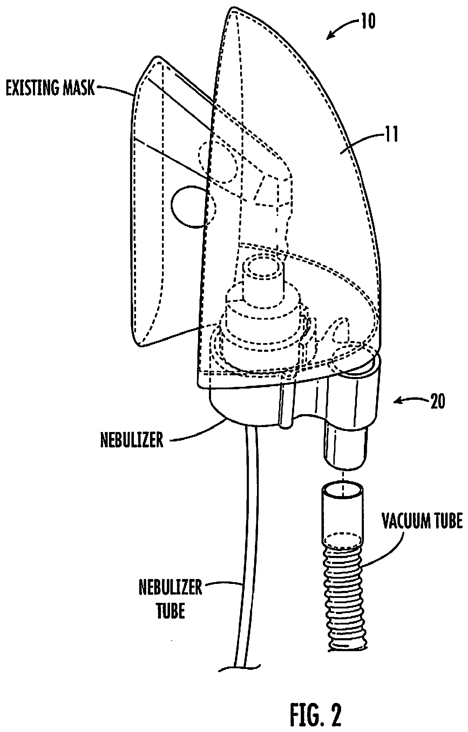

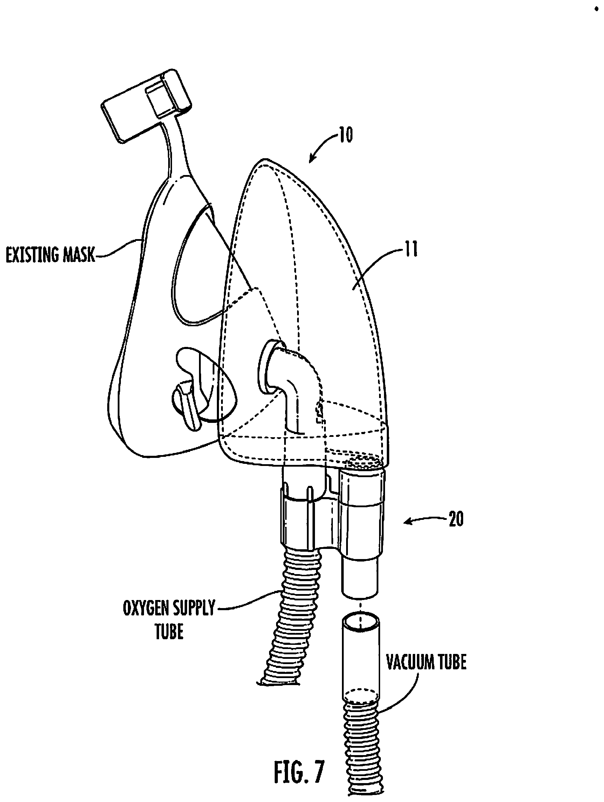

As shown at least in the illustrative embodiments of Figures 1-4, 6-10 and 12, the vacuum shield assembly 10 comprises a shield body 11. The vacuum shield assembly 10 also generally comprises a retaining assembly 20. The retaining assembly 20 is generally connected to the shield body 11 as well as to a vacuum tube. As used herein, the term "vacuum tube" refers to a conduit, hose, or other related structure that may convey air from a patient and/or mask to another location, and which may be connected to a negative pressure vacuum. For example, the vacuum tube 40 may comprise a 22 millimeter hose, which may comprise a length of about 8 feet. As shown at least in figures 2 and 7, the retaining assembly 20 may be used to interconnect the shield body 11 to a vacuum tube. The structure of the retaining assembly 20 should define a fluid communication between an inside of the shield body 11 and the vacuum tube. As such, the shield body 11 may create a negative pressure on an interior thereof to remove the air between the medical mask, the face and/or head of the patient, and the interior or inside of the shield body 11. It is contemplated that a patient that is wearing a BIPAP or CPAP mask, or a nebulizing mask, be able to exhale through the mask, and that at least a portion of this exhaled air may be captured by the negative pressure generated by the shield body 11 and the vacuum tube.

As is shown in Figures 1-2 and 6-7 the retaining assembly 20 may also be used to connect the shield body 11 and/or vacuum tube to an oxygen supply tube and/or a nebulizing unit or component thereof. Shield bodies of different sizes may be attached to a retaining assembly 20, for example, by inserting a connecting portion 18 into an upper section 21" of the retaining component 21, or to the retaining component 21 directly, which will be explained later. As such, it may be possible to switch between shield bodies of different sizes according to a specific need, e.g., air suction, nebulization, etc., and/or geometrical constraints, e.g., the size of the head of the patient.

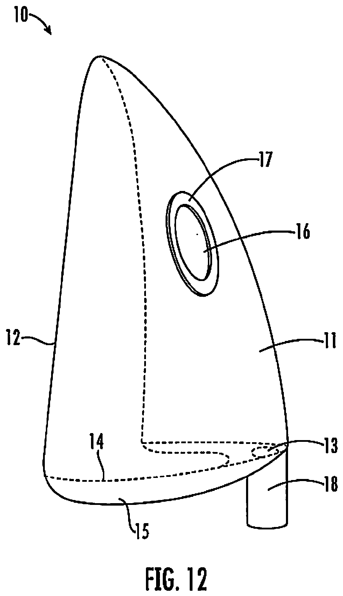

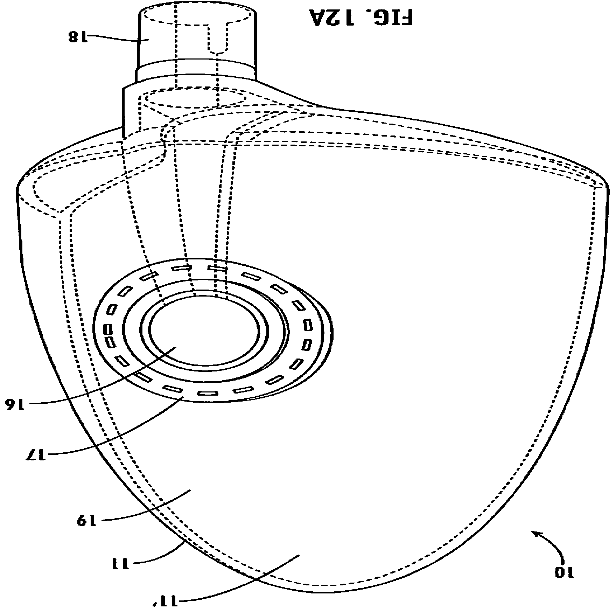

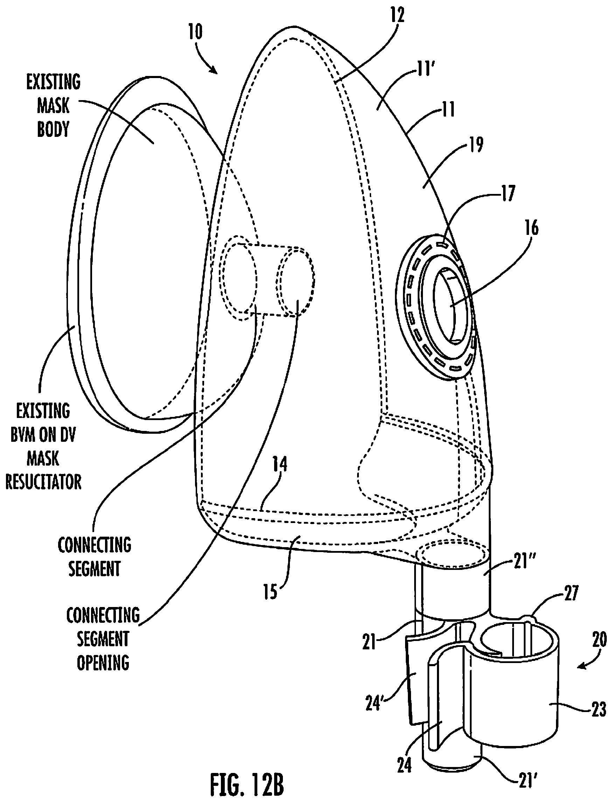

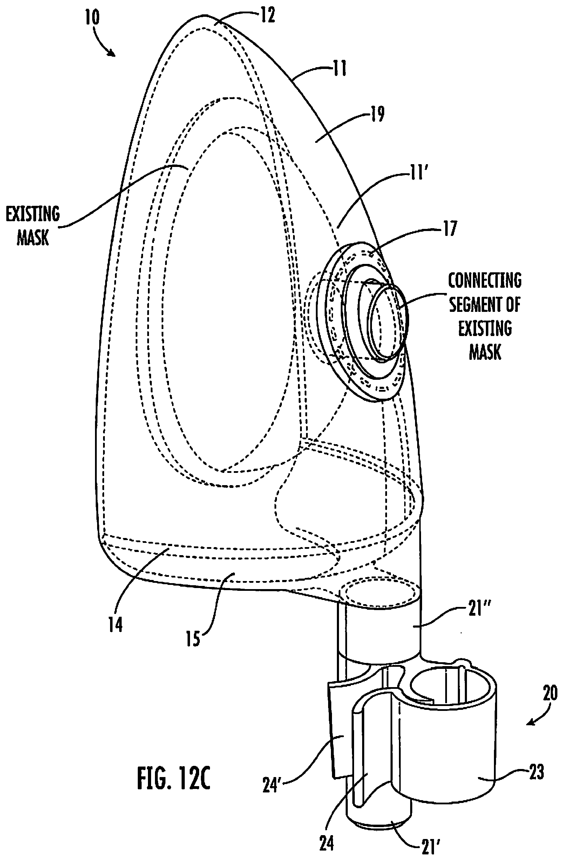

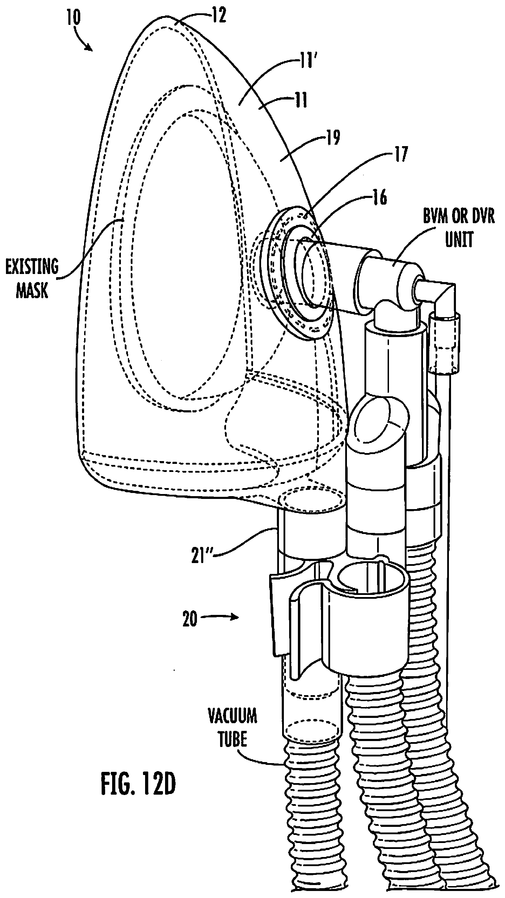

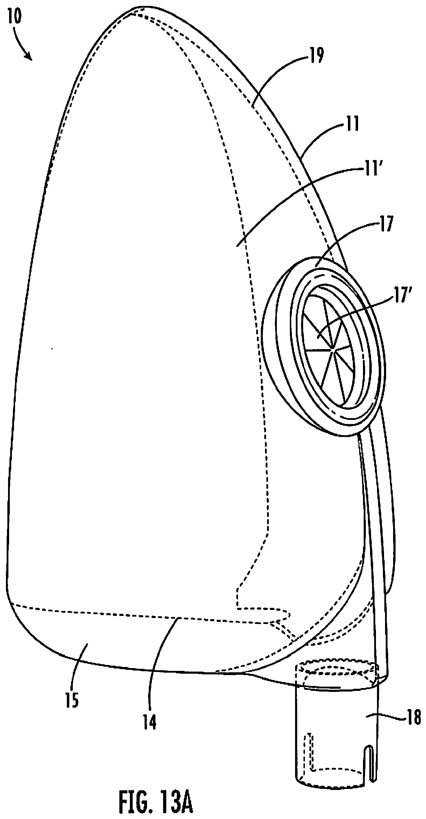

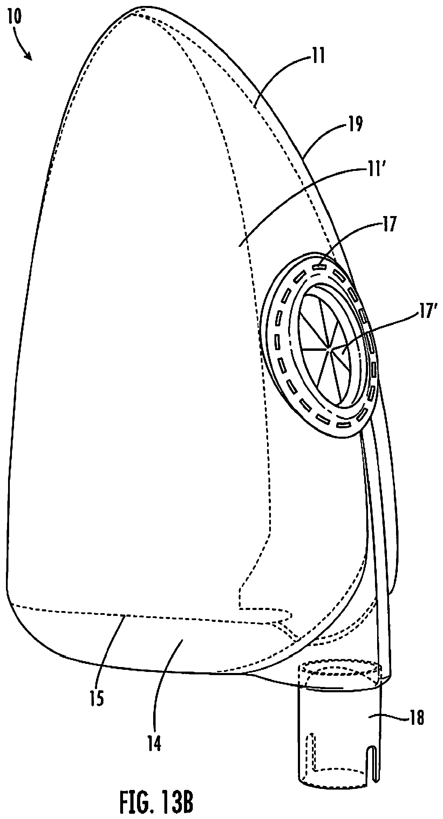

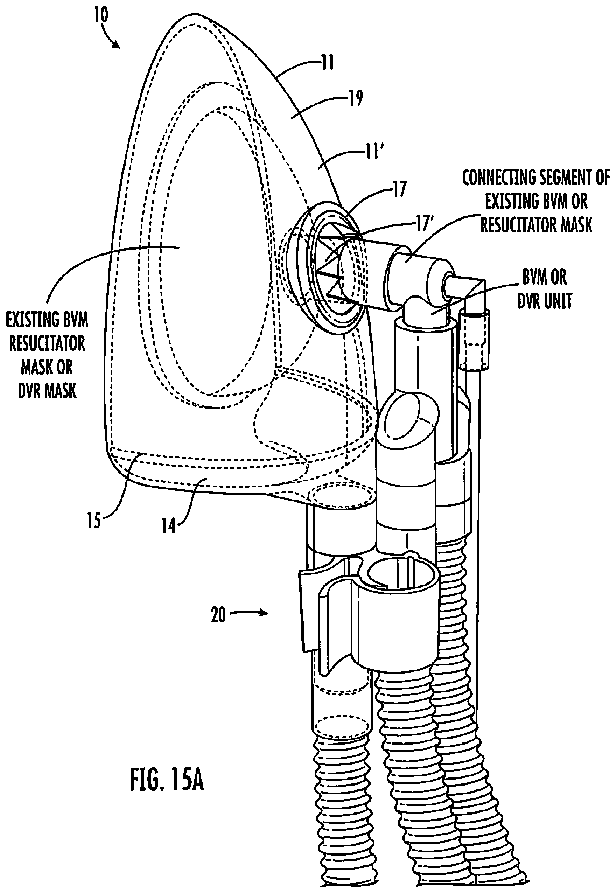

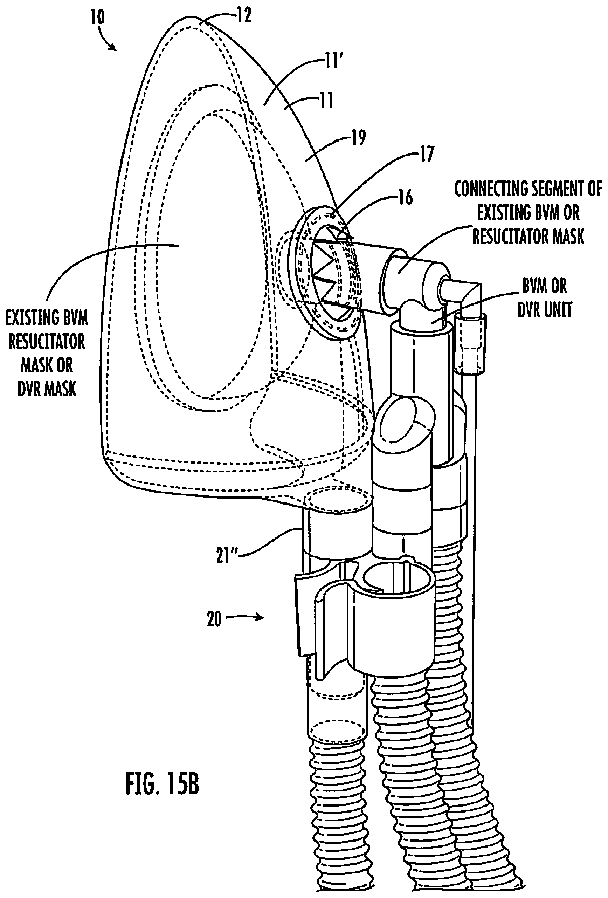

With reference to Figures 12A-15B, the shield body 11 according to the inventive vacuum shield assembly 10 may be provided with a second opening 16 configured and dimensioned to accommodate a medical mask. With specific reference to Figures 12B-12D and 14A-15B, the second opening 16 may be configured and dimensioned so that a connecting segment of an existing medical mask, e.g., a demand-valve resuscitator (DVR) mask or a bag-valve-mask (BVM) resuscitator mask, may be inserted there through, e.g., as shown in Figs. 12D, 15A and 15B. Furthermore, the shield body 11 may be provided with a vacuum attachment 17, which may at least partially define or otherwise form a seal between an outer surface 11' and an inner surface 11" of the shield body 11. Also, the second opening 16 and/or vacuum attachment 17 may be operatively configured and dimensioned to substantially define a seal between the second opening 16 and the connecting segment of the medical mask. Accordingly, the vacuum attachment 17 may comprise a grommet component or grommet seal. However, this is not necessarily limiting as other configurations of the vacuum attachment 17 are also possible.

As represented in at least Fig. 15A, a grommet seal may be co-molded to the shield body 11. This is advantageous as it may at least partially reduce the time, effort, and/or expense involved in manufacturing a vacuum shield assembly 10 with a vacuum attachment. Further, a grommet seal co-molded to the shield body may provide for a robust construction, which is also advantageous. Alternatively, as represented at least in Figure 15B, a grommet seal may be inserted into the shield body, which is referred to as an insert-molding. Further, the vacuum attachment 17 may comprise a variety of materials, including, but not necessarily limited to silicone, rubber, plastics, elastomeric polymers, seals, sealants, and/or other related structures. As such, the second opening 16 may at least partially allow an operative communication between the existing mask, i.e., BVM or DVR, through an interior of its connecting segment, and the underlying BVM or DVR unit. It is contemplated that the opening 16 permit at least a fluid communication between the existing mask and the BVM or DVR unit, i.e., via the interior of the connecting segment of the mask, which passes through the second opening 16.

With reference again to Figures 12A-15B, the second opening 16 may be disposed on the shield body 11 at a location that corresponds to the location of the medical mask. Generally, during some BVM and/or DVR procedures some air may leak between the face of the patient and the mask, which is attached to the patient, for example, as the patient inhales or exhales air. In such BMV and/or DVR procedures, the inventive vacuum shield assembly 10 is intended to capture exhaled air that may leak out of the BVM and/or DVR mask. As such, the second opening 16 may be disposed substantially around a middle section of the shield body 11 and/or above the first opening 13. This would allow for placement of the vacuum shield assembly 10 on a location that corresponds to location of the connecting segment of the medical mask. Moreover, the second opening 16 and/or vacuum attachment 17 may be disposed on a height along the shield body 11 that corresponds to the approximate location of the existing medical mask. As may be appreciated from the illustrative embodiments as shown in Fig. 15A and 15B, exhaled air may exit through the existing mask, i.e. through opening 13 of the shield body 11. In addition to, or in lieu of this, exhaled air may also exit through a vacuum tube operatively connected to the existing mask and connecting segment, which passes through the second opening 16.

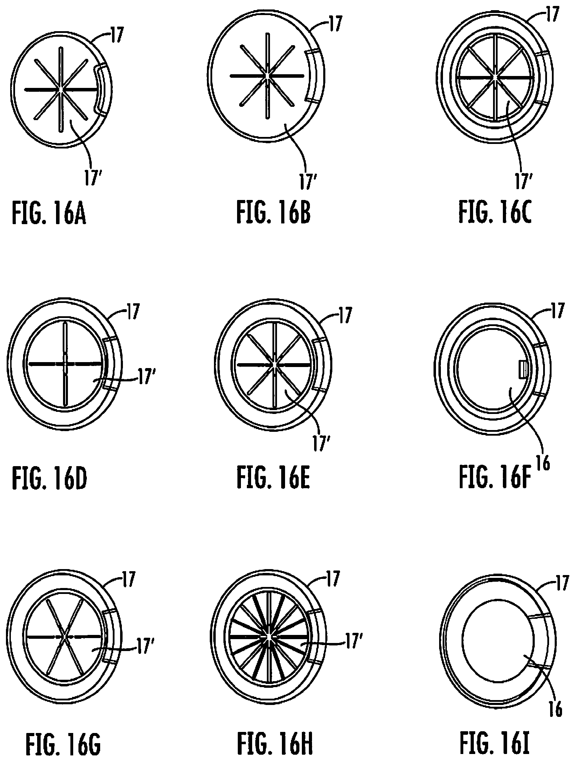

As is perhaps best shown in Figures 16A-16I, features of the present invention comprise providing a vacuum attachment 17 comprising a grommet configuration. As shown in Figures 16A-16I, the vacuum attachment 17 may comprise a substantially circular shape configured to correspond to the diameter and/or size of the second opening 16. For example the diameter of an outer perimeter or recessed portion of the vacuum attachment 17 may be configured to correspond to the dimension and/or size of the second opening 16. Additionally, the circular shape of the vacuum attachment 17 may be configured and dimensioned to correspond to the diameter and/or size of the connecting segment of the medical mask, for example around an inner perimeter of the vacuum attachment 17. As shown in Figures 16F and 16I, a vacuum attachment 17 may be provided comprising a grommet configuration with an aperture. As such, when the vacuum attachment 17 is disposed around the second opening 16, the aperture of the vacuum attachment 17 permits a fluid communication between the outer surface 11' and inner surface 11" through the second opening 16. As shown in Figure 16F, the vacuum attachment 17 may comprise a tear away recessed pocket. Conversely, as shown in the illustrative embodiments of Figures 16A-16E and 16G-16H, the vacuum attachment may comprise a grommet configuration that creates a cover around the interior perimeter of the vacuum attachment 17. The cover may comprise a plurality of adjacently disposed segments 17', which may be collectively structured to form a substantially flat surface in an inoperative disposition of the connecting segment of the medical mask, i.e., when the connecting segment is not inserted around the second opening 16. The plurality of adjacently disposed segments 17' may be collectively structured to bend at least in an opposite direction to the movement of the connecting segment of the medical mask.

As is shown in the illustrative embodiments of Figures 15A and 15B, once the connecting segment of the medical mask is inserted through the second opening 16, the plurality of adjacently disposed segments 17' may bend towards the outer surface 11' allowing the connecting segment to pass there through. Conversely, if the connecting segment is removed, the plurality of adjacently disposed segments 17' may return to their natural and/or initial position, forming once again the cover around the aperture of the vacuum attachment 17. As such, a vacuum shield assembly 10 according to the present invention may be used in connection with one BVM and/or DVR procedure, then later removed, and used in a subsequent BVM and/or DVR procedure. The vacuum shield assembly 10 may also be used in connection with a procedure that uses the second opening 16, i.e., a BVM and/or DVR procedure, and may later be used in a subsequent procedure that does not use the second opening 16, or vice versa. Or alternatively, a vacuum shield assembly 10 comprising a second opening 16 may also be used in connection with a procedure that does not need the second opening 16. Thus, a vacuum shield assembly 10 comprising a second opening 16 may be attached to a medical mask that is not a BMV or DVR mask. As such, the plurality of adjacently disposed segments 17' may naturally form a cover, which should essentially function as a seal between the outer surface 11' and inner surface 11". Said differently, the plurality of adjacently disposed segments 17', in their natural position, should at least partially reduce leakage of exhaled air from the inner surface 11" to the outer surface 11'. As such, exhaled air may be retained on an interior of the shield body 11 and removed via the connecting portion 18 and/or oxygen tube.

As is also seen in Figures 16A-16E and 16G-16H, each one of the plurality of adjacently disposed segments 17' may comprise a substantially triangular shape. As such, when disposed around the inner perimeter of the vacuum attachment 17, they may substantially define a cover. By way of example, the plurality of adjacently disposed segments 17' comprising a substantially triangular shape may comprise four segments, e.g., Fig. 16D, six segments, e.g., Fig. 16G, eight segments, e.g., Fig. 16A, 16B, 16C, 16E and 16H, or even more than eight segments. Furthermore, as shown in Figure 16H, the plurality of adjacently disposed segments 17' may comprise reinforcement ribs. Also, the illustrative embodiments of Figures 16D-16E and 16G may comprise a top flush relief ring, or a recessed pocket as is shown in Figures 16C-16H. As shown in Figures 16A-16B, other possible configurations of the plurality of adjacently disposed segments comprises a top flush configuration without a relief ring.

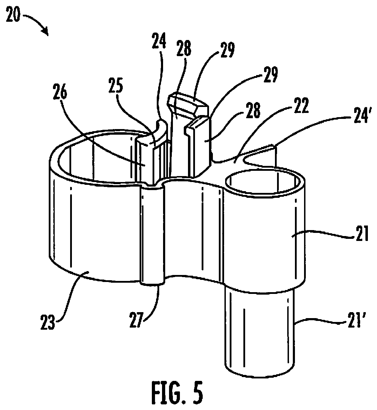

As is perhaps best shown in Figures 5-6, and as mentioned below, the inventive vacuum shield assembly 10 comprises a retaining assembly 20. As shown in Figures 1 and 6, the retaining assembly 20 may be oriented towards the face of a patient, such that it may be used to attach the shield body 11 to an existing vacuum tube or other related component. Various connecting mechanism of the retaining assembly 20 may be implemented to connect it to the shield body 11, vacuum tube, oxygen supply tube or nebulizing unit. Said differently, the retaining assembly 20 may be used to interconnect the shield body 11 to the vacuum tube and the oxygen supply tube, an existing nebulizing unit and/or mask, or an existing BIPAP or CPAP mask. As an example, the retaining assembly 20 may comprise clamps or connecting arms. Other mechanisms of the retaining assembly 20 are also within the scope of the present invention and may comprise adhesives, connecting bands, snap-on mechanisms, magnets, or another related connecting mechanisms.

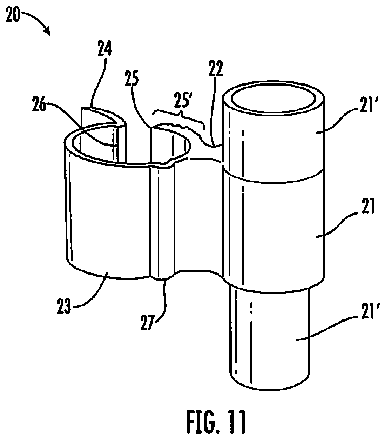

As seen in the illustrative embodiments of Figures 5 and 11, the retaining assembly 20 may comprise a retaining frame 23 connected to a retaining component 21. As mentioned above, a connecting portion 18 of the vacuum shield assembly 10 may be configured and dimensioned to correspond to the size of a retaining component 21 of the retaining assembly 20. As may be appreciated from Figure 11, sometimes it may be beneficial to provide for a height adjustment for the point of connection between the connecting portion 18 of the shield body 11 and the retaining assembly 20. In such embodiments, the retaining component 21 may be provided with an upper section 21", which may at least partially raise the position of the shield body 11 relative to the point of attachment of the retaining assembly 20 to the oxygen supply tube or other related component of the existing mask.

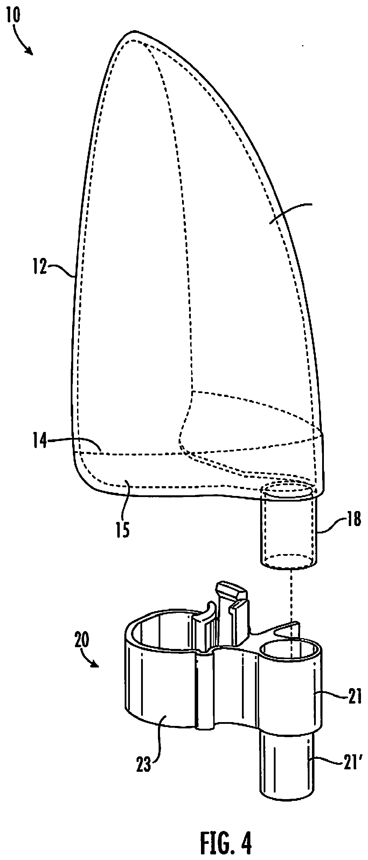

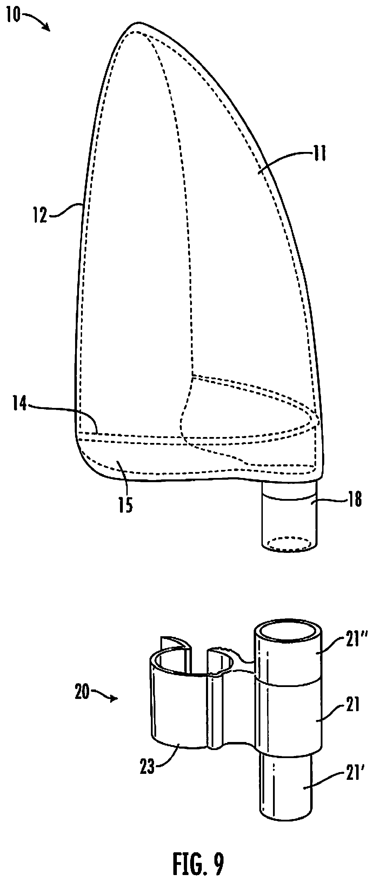

Additional features of the present invention comprise providing a shield body 11 that may be configured and dimensioned to correspond to the geometry and/or size of the head and/or face of patient and/or the existing mask and its components. It is within the scope of the present invention that when the shield body 11 is disposed against the existing mask that a substantial portion of the edge 12 at least partially surround the existing mask. That is, the shield body 11, including the edge 12 of the perimeter, should define a profile or area that is at least equal to or even greater than the profile or area of the existing mask. As such, exhaled air from the patient will be retained on an inside of the shield body 11, including above a lower segment 15. As an example, as is shown at least in Figures 4 and 9-10, the edge 12 may comprise a semi-ovoidal configuration. As is perhaps best shown in Figures 4 and 9, the edge 12 may also define a substantially flat side profile of the shield body 11. However, the shield body 11 may comprise other shapes to correspond to the shape of the existing mask. The lower segment 15 may be configured and dimensioned to accommodate the size and/or geometry of an oxygen supply tube of a BIPAP or CPAP mask, or the size and/or geometry of a nebulizer unit and/or components thereof.

The illustrative embodiment of Figures 6-9 and 11 show a retaining assembly 20 comprising a retaining component 21 and an upper section 21" thereof which provides for a vertical offset. The length of the upper section 21" may be configured and dimension according to preferences, type of existing mask, intended application, amount of height adjustment needed for the shield body 11, etc. These illustrative embodiments, both of the retaining component 21 and the upper section 21" comprise a substantially cylindrical configuration with approximately the same diameter. Conversely, as is in the illustrative embodiments of Figures 1-5, the retaining assembly 20 may be provided with a retaining component 21 without an upper section 21". It is within the scope of the present invention that the connecting portion 18 of the shield body 11 be attachable to the retaining component 21 and/or upper section 21" thereof. For example, the connecting component 18 may comprise a substantially cylindrical configuration, which may be configured and dimensioned to correspond to the size of an inside of a cylindrical retaining component 21 and/or upper section 21". Further to this example, and as is perhaps best shown in Figures 4 and 9, the outer diameter of the connecting component 18 may be at least partially smaller than the inner diameter of the retaining component 21 and/or upper section 21", such that the connecting component 18 may be inserted into the retaining component 21 and/or upper section 21". In at least one embodiment the retaining component 21 and upper section 21" may comprise the same diameter. Additionally, in such embodiments, both diameters of the connecting component 18, retaining component 21 and/or upper section 21" may be configured and dimensioned to enable a frictional resistance between corresponding surfaces such that the shield body 11 may be connected to the retaining assembly 20, and further, so that it may remain in place during periods of operation or use of the inventive vacuum shield assembly 10.

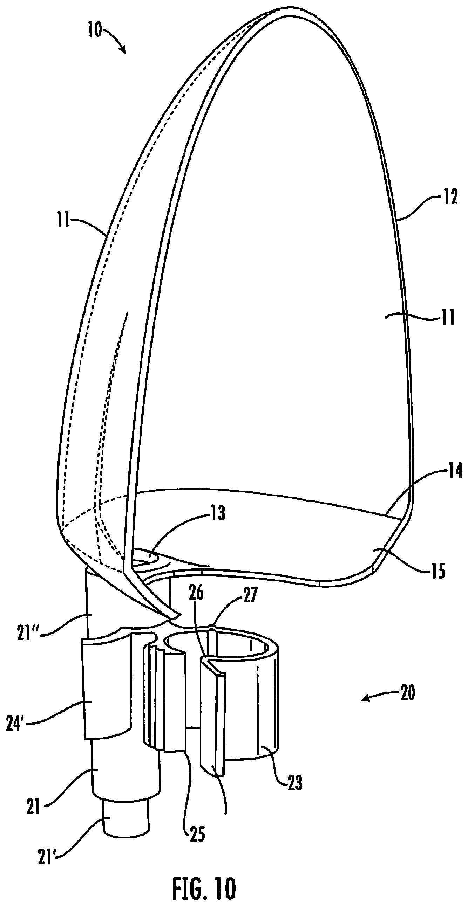

As may be perhaps best shown in the illustrative embodiments of Figures 2-5 and 7-11, the retaining component 21 of the retaining assembly 20 comprises a lower section 21'. The inside of the lower section 21' of the retaining component 21 should be disposed in fluid communication with the inside of the retaining component 21, the inside of the connecting portion 18 of the shield body 11, the inside of the upper section 21" of the retaining component, and/or the inside of the vacuum tube. Additionally, the lower section 21' of the retaining component 21 may be configured and dimensioned for attachment of the vacuum tube. By way of example only, the lower section 21' of the retaining component 21 may be provided with an outer diameter that is at least partially smaller to an inner diameter of the vacuum tube. As such, the vacuum tube may be attached to the outside of the lower section 21' of the retaining component 21, and may be disposed in fluid communication with an inside of the lower section 21' of the retaining component 21, the inside of the retaining component 21, an inside of the upper section 21" of the retaining component 21, and/or an inside of the connecting portion 18. This should enable a fluid communication between the vacuum tube and the shield body 11, including on an interior or inside thereof, which is perhaps best shown in Figure 10. As such, activation of the vacuum tube will result in a negative pressure around the inside of the shield body 11. Such a negative pressure will result in at least a partial removal of the air on the inside of the shield body 11 and/or the surrounding area.

With reference to at least Figures 5 and 11, and as mentioned above, the retaining assembly 20 may be provided with a retaining frame 23. The retaining frame 23 may be connected to the retaining component 21, for example, via a transition structure 22. The retaining frame 23 is intended to attach the retaining assembly 20, and consequently the shield body 11 and vacuum tube, to a component of the existing mask. For example, such a component of the existing mask may include an oxygen supply tube of a BIPAP of CPAP mask. Also as an example, such a component of the existing mask may also include a nebulizing unit or a portion or component thereof. The retaining frame 23 should comprise an inner area, which may be selectively adjusted to securely retain the oxygen supply tube or nebulizing unit or component thereof. For example, the retaining frame 23 may comprise a substantially cylindrical configuration and/or two segments which may be connected to one another. A first closing structure 25 and a second closing structure 26 may be provided and may be cooperatively configured to form a closing mechanism or engagement that retains the oxygen supply tube or nebulizing unit. Also as an example, the first closing structure 25 and/or second closing structures 26 may be provided with a closing mechanism or related components that may enable such closing mechanism or engagement.

In the illustrative embodiments of Figures 1-11, a first closing structure 25 may be provided with a snap component whereas a second closing structure 26 may be provided with serrations 25'. The snap component and the serrations 25' may be cooperatively configured with one another to form a mating engagement, and allow a user or medical practitioner to selectively increase or decrease the inner area of the retaining frame 23. For example, the snap may be selectively disposed in any one of a plurality of serrations 25' along the length of one of the segments of the retaining frame 23. As used herein, a "snap" mechanism generally refers to a single-snap mechanism, or a multi-snap mechanism, i.e., an adjustable mechanism that may be selectively disposed into various size settings. As such, one single retaining assembly 20 may be used in connection with various oxygen supply tubs of different sizes and/or nebulizer units of different sizes. To further assist the user or medical practitioner in adjusting the inner area or opening of the retaining frame 23, one or more flaps 24 and/or 24' may be provided. The flaps 24 and/or 24' may be disposed or otherwise formed on the segments of the retaining frame 23, including around the first closing structure 25 and/or second closing structure 25. The flaps 24 and/or 24' may extend along the height of the retaining frame 23 and/or may comprise a size that corresponds to the size of the thumbs and/or fingers of a user or medical practitioner. Thus, selective movement of the flaps 24 and/or 24 will result in a corresponding movement of at least one of the segments of the retaining frame 23, and consequently movement of a corresponding closing structure 25 and/or 26. Although a retaining assembly 20 may be provided comprising two flaps 24 and 24', it is also possible to provide a retaining assembly 20 comprising only one flap 24 or one without any flaps.

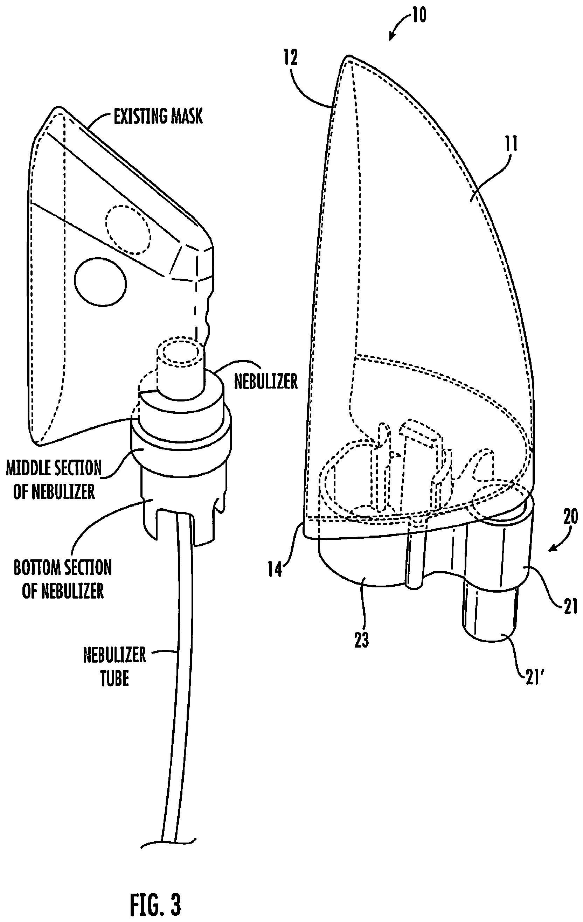

As is perhaps best show in in Figure 5, the retaining frame 23 may be provided with at least one retaining segment 28 configured to at least partially retain the nebulizing unit. For example, as shown in the illustrative embodiment of Figure 3, two retaining segments 28 may be used to at least partially retain a middle section of a nebulizing unit. Further, each retaining segment(s) 28 may comprise latch 29 disposed around an upper end thereof. The latch(es) 29 may be configured to hold the top of the middle section of the nebulizing unit in place and at least partially reduce its movement in the vertical direction. As is also shown in the illustrative embodiment of Figure 3, and also in other embodiments, the retaining frame 23 may be provided with a substantially cylindrical or semi-cylindrical configuration. Such configuration is advantageous to retain or otherwise attach the retaining assembly 20 to substantially cylindrical nebulizers or oxygen supply tubes.

With reference now to at least Figures 1-3 and 6-8, features of the present invention comprise providing a vacuum shield assembly 10 with a shield body 11 and a retaining assembly 20 collectively disposable into and out of an operative position and an inoperative position. As used herein, the "inoperative position" refers to a position of non-use of the vacuum shield assembly 10, and may include a storage position, an inactive position, a position where the vacuum shield assembly is not connected to external components, e.g., an oxygen supply tube, vacuum tube, nebulizer unit, face or head of a patient, etc. Conversely, as used herein, the "operative position" refers to an operational or otherwise active positon of the vacuum shield assembly 10. In the operative position, the shield body 11 should be connected to and disposed in fluid communication with the retaining assembly 20. As is shown at least in Figures 1-3 and 6-8, in the operative position, an interior or inside of the shield body 11 should be oriented toward the existing mask, which should already be disposed on the face and/or head of the patient. In the operative position, the vacuum tube, and/or connected vacuum source, should exert a negative pressure, which should result on a corresponding exerted negative pressure around the shield body 11 and the surrounding area. It is contemplated that in the operative position, the negative pressure exerted around the inside or interior of the shield body 11, and/or above the lower segment 15, should be sufficient to at least partially extract the exhaled air form the patient. Also, the lower segment 15, along with the interior or inside of the shield body 11, is intended to at least partially retain exhaled air between the face of the patient and/or existing mask, and the shield body 11. As such, movement of the exhaled patient air outside of the area surrounding the shield body 11 may be at least partially reduced, such that, the negative pressure of the vacuum tube, should result on an efficient removal of the exhaled air.

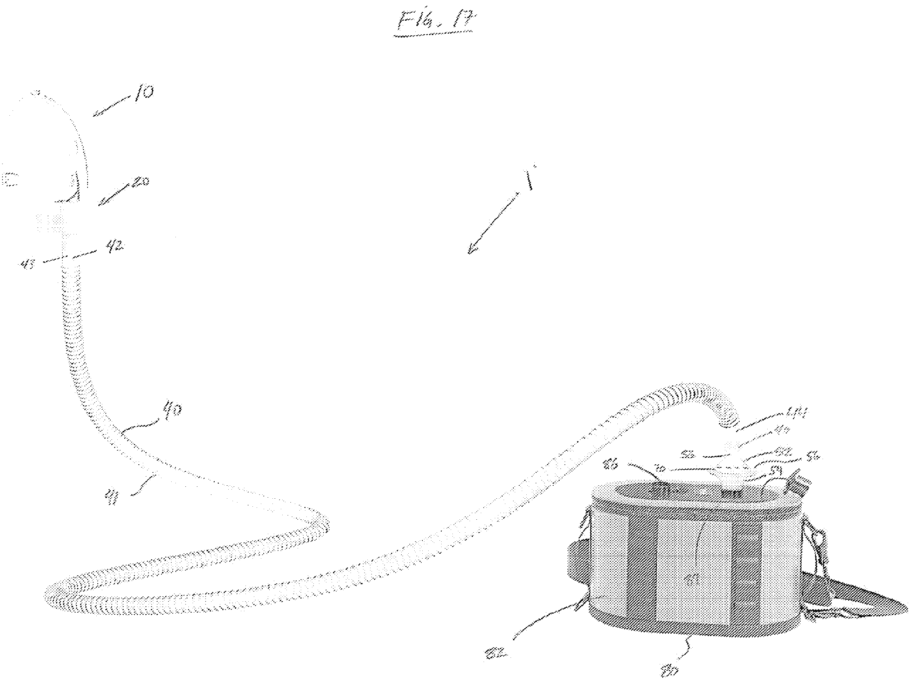

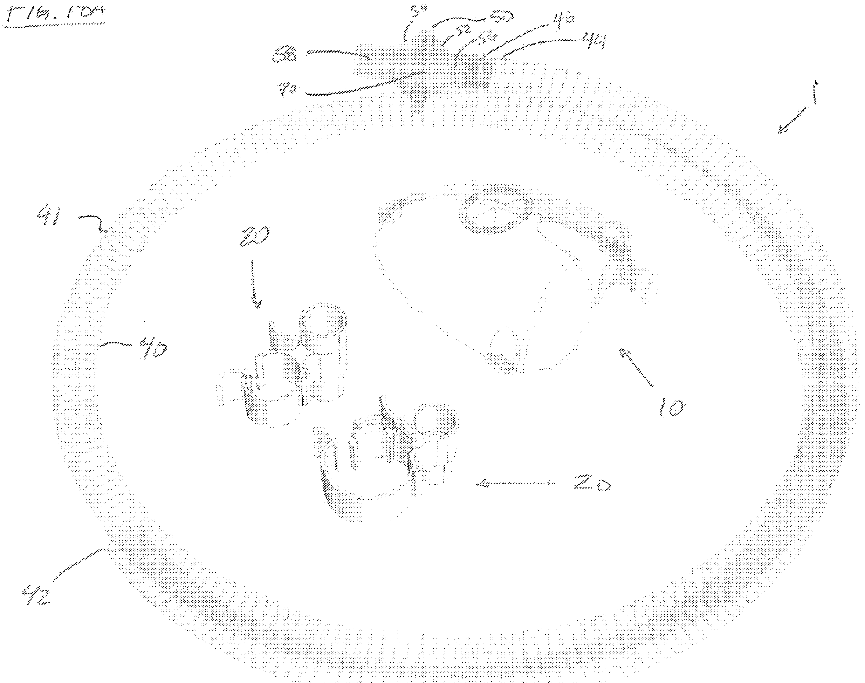

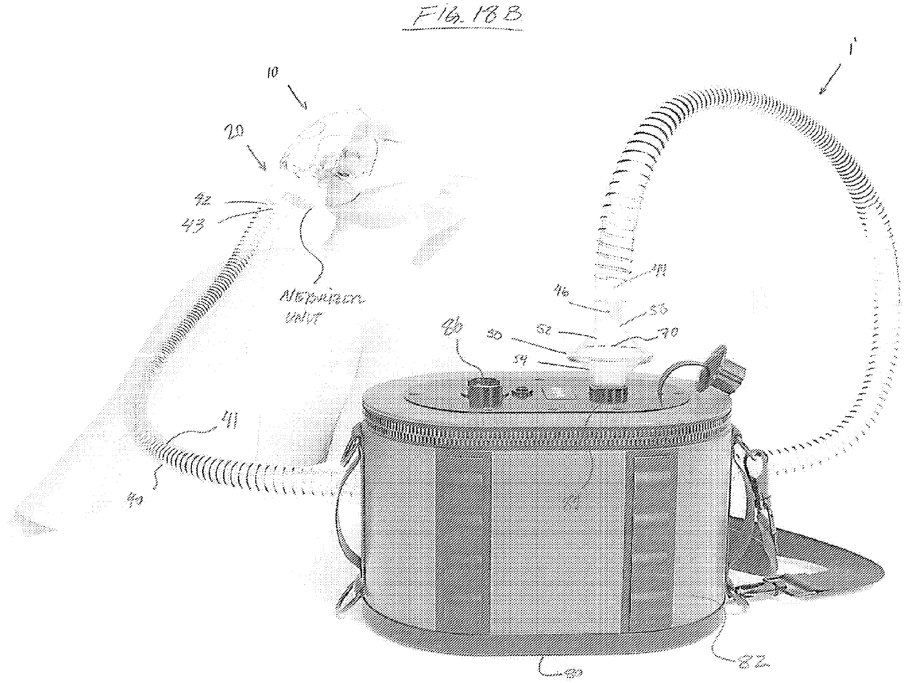

With reference to Figures 17-19D, further embodiments of the present invention relate to a system 1' configured to remove exhaled air from a patient. Generally, the system 1' according to the present invention is configured to remove exhaled air from a patient wearing a medical mask as defined herein, but may also be used on patients not wearing a medical mask as the various components of the system 1' may be at least partially disposed on the patient directly. With specific reference to at least Figures 17 and 18, the system 1' generally comprises at least a vacuum shield assembly 10 as defined herein, a retaining assembly 20 as defined herein, a vacuum tube 40 and a vacuum unit 80. In embodiments of the present invention where the shield body 11 may be disposed directly on a patient not wearing a medical mask, a retaining assembly 20 may not be necessary as the vacuum tube 40 may be disposed directly on the shield body 11. As used herein, a vacuum unit 80 refers to a vacuum device, which may be motor operated, and which may be disposed in fluid communication with a vacuum tube 40, i.e., a hose, or other flexible or expandable hollow elongated component, and which may exert a negative pressure. It is contemplated that a shield body 11 of the shield assembly 10 also be disposed in fluid communication with the interior of the vacuum tube 40. The negative pressure of the vacuum unit 80 should be transferred through the vacuum tube 40, and to an interior face of the shield body 11, i.e., the side that faces the patient. As such, the shield body 11 essentially acts as a vacuuming device that is capable of at least partially removing exhaled air around the face of the patient. As used herein, the space defined by the interior face of the shield body 11 as well as the face of the patient, including when wearing the medical mask, is defined as an enclosure zone 19.

With reference to at least Figure 18A, the system 1' according to the present invention may comprise a retaining assembly 20 configured to retain the vacuum tube 40 and a component of the medical mask as defined herein, i.e., an oxygen supply tube of a BIPAP of CPAP mask or a nebulizing unit or a portion or component thereof. Further, the vacuum tube 40 may comprise a body 41 of a flexible, or elastic material that may at least partially bend, twist, move or otherwise conform to geometric constraints. The vacuum tube 40 may comprise a proximal end 44 that connects to the vacuum unit 80 around connecting end 36, and a distal end 42 that connects to the shield body 11 around a connecting end 46.

With reference to at least Figures 18A-18B, a filter case assembly 50 may be disposed or integrally formed on the vacuum tube 40 around the proximal end 44. The filter case assembly 50 may comprise a top segment 52 and a bottom segment 54 which may be operatively connected to one another. In other words, the top segment 52 and bottom segment 54 may form a mating engagement with one another in a secured position, i.e., once a filter 70 has been placed between them. It is contemplated that the mating engagement between top segment 52 and bottom segment 54 not be a permanent mating engagement such that the top segment 52 and bottom segment 54 may be removably connected to one another to insert and/or remove a filter in the area where they engage. The diameters of the top segment 52 and/or bottom segment 54 should correspond to one another and should be configured and dimensioned to accommodate the diameter and/or size of a filter 70, which may comprise an air filter such as an ultra-low particulate air (ULPA) filter. The filter 70 may also comprise a high efficiency particulate air (HEPA) filter. As such, the diameters of the top segment 52 and/or bottom segment 54 may be larger than the diameter of the vacuum tube 40, but this is not strictly necessary. When the top segment 52 and the bottom segment 54 are forming a mating engaged, this should restrict placement or otherwise movement of a filter 70 disposed therein. Furthermore, the top segment 52 and/or upper segment 54 may be provided with conical or semi-conical shapes. This may be done to at least partially facilitate airflow through the vacuum tube 40 and into the vacuum unit 80 and/or to otherwise at least partially reduce the likelihood of a bottleneck effect around the area where the filter 70 is disposed. Consequently, air captured around a distal end 42 of the vacuum tube will pass through the filter 70 before entering the vacuum unit 80, at least partially reducing contaminants and/or other infectious particles.

With reference to at least Figures 17 and 18B, and as mentioned above, the system 1' according to the present invention comprises a vacuum unit 80. The vacuum unit 80 should provide for a portable solution of creating a negative vacuum pressure, at least around its first opening 84 where the vacuum tube 40 will be connected. The vacuum unit 80 may comprise a housing 82 with a first opening 84 and a second opening 86. The first opening 84 is generally configured for attachment of the vacuum tube 40, for example, around a proximal end 44 thereof. The second opening 86 is generally configured for captured air to exit outside of the housing 82. That is, air collected form the enclosure zone 19 that passes through the first opening 84 and into the interior of the housing 82, should be able to exit outside of the housing 82 through the second opening 86. Alternatively, other means of air escape may be provided on the housing, and may include slots or vents, including disposed on the sides. In addition, the interior of the housing 82, which is generally a chamber, may be provided with other filtering means to further remove contaminants and/or infections particles form the air captured around the enclosure zone 19. Also, a top cover of the housing may be removable from the rest of the housing 82 to access any components thereof, which may include a battery-operated vacuum with a motor, additional filtering components, etc. By way of example only, a battery-operated vacuum motor of 110V or similar, may be provided on an inside of the housing 82.

Specific to the system 1' according to the present invention, the vacuum unit 80, vacuum tube 40 and shield body 11 may be collectively disposable into and out of an operative orientation and an inoperative orientation. The operative orientation comprises the vacuum unit 80 activated and exerting a negative pressure on an inside of the shield body 11, i.e., around the enclosure zone 19, to at least partially remove exhaled patient air. The inoperative operation comprises periods of non-operation of the system 1', including when the vacuum unit 80 is inactive. Also, the shield body 11, the vacuum tube 40 and the retaining assembly 20 may be collectively disposed into and out of an operative position and an inoperative position. With reference to Figure 18B, the operative position generally comprises the shield body 11 at least partially attached to the head of the patient and the vacuum tube 40 operatively connected to the shield body 11. The operative position may also comprise the vacuum tube 40 and the component of the medical mask disposed onto the retaining assembly 20. The operative orientation may also comprise disposing the shield body 11, the vacuum tube 40 and the retaining assembly 20 into the operative position and the vacuum unit 80 being activated to exert a negative pressure on the inside of the vacuum tube 40 as well as the interior of the shield body 11, at least partially removing exhaled air from the patient around the enclosure zone 19. In at least one embodiment of the system 1' according to the present invention, the system 1' is capable of removing at least 93% of exhaled particles having a size of 0.5 micron. In order to achieve this, the system 1' should be capable of delivering a negative pressure of at least 240 liters per minute, measured around the interior of the shield body 11, and in some embodiments up to about 280 liters per minute. In turn, this at least partially achieves a re-breathing or re-inhalation reduction of up to 6%, while at the same, given the geometry of the components of the shield body 11, does not substantially reduce the amount of inhaled oxygen, i.e., from an oxygen supply tube, or nebulizer particles. In some embodiments the amount of nebulizer particles provided to the patient is maintained, and in other embodiments, even increased.

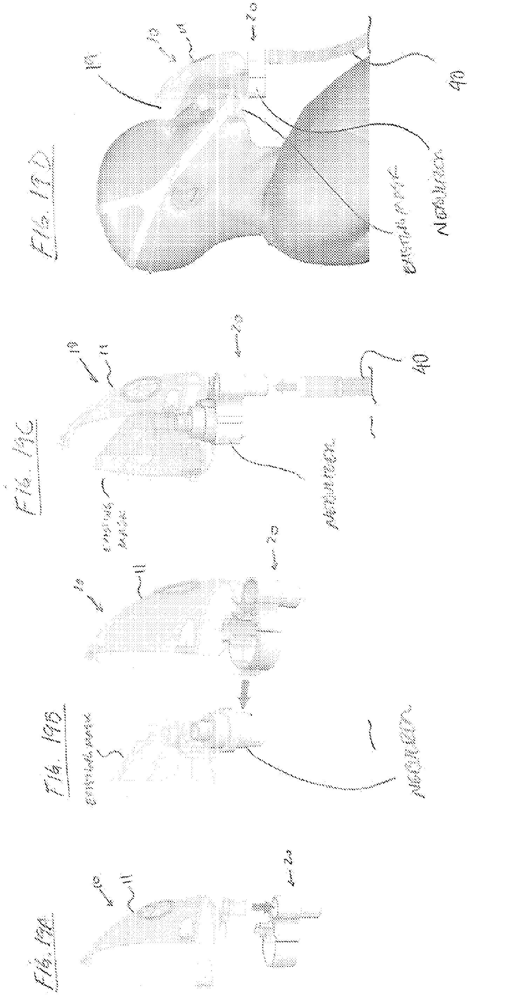

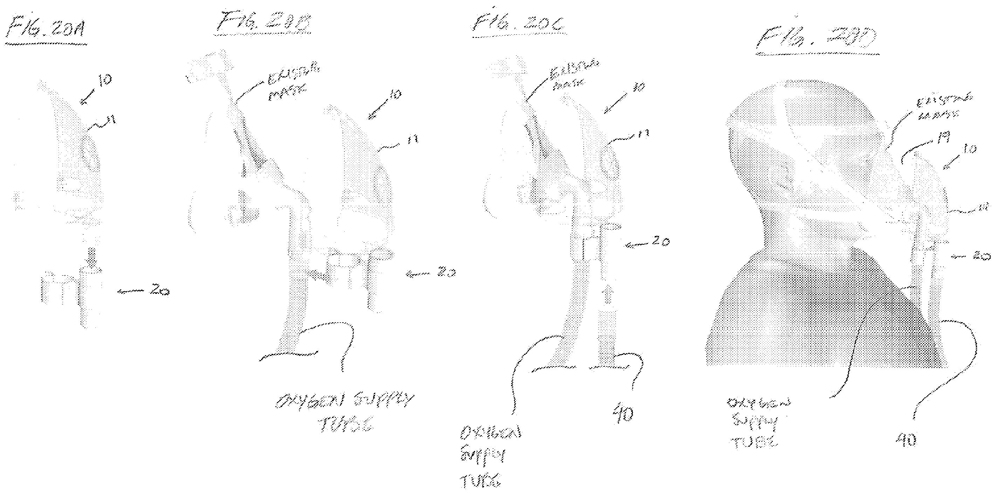

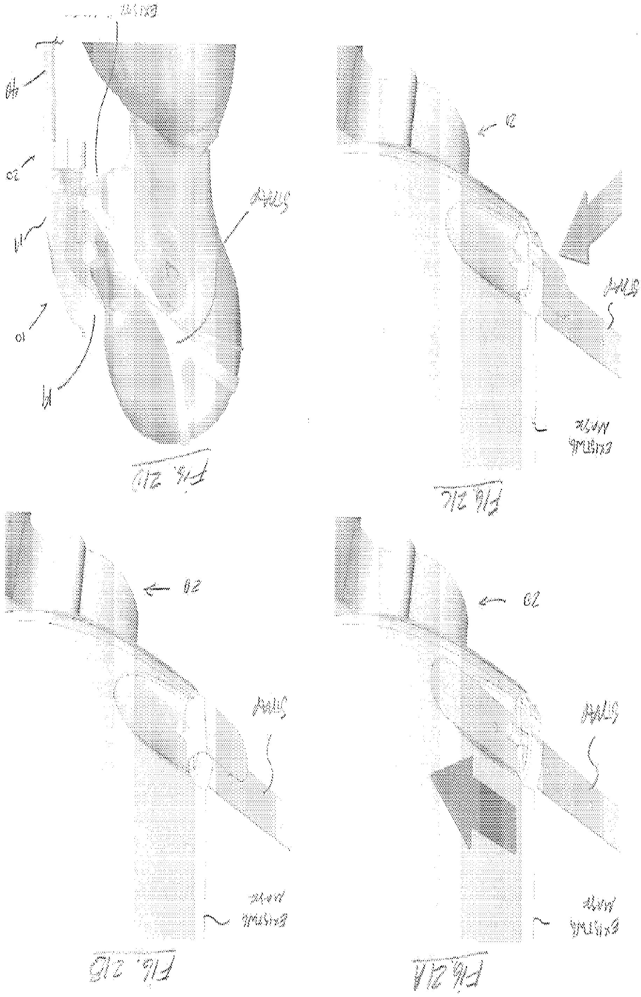

With reference now to at least Figures 19A-21D, and as mentioned above, various components of the innovative system 1' may be disposed into an operative position as shown in Figures 19D, 20D and 21D. The shield body 11 may be connected to the retaining assembly (Figures 19A and 20A). Thereafter, the retaining assembly 20 may be connected to an oxygen supply tube of an existing mask (Figure 20B), or to a nebulizer (Figure 19B). Thereafter, the vacuum tube 40 may be connected to the retaining assembly 20 (Figures 19C and 20C). In some embodiments where a medical mask or existing mask is provided with an adjustable strap. The strap may be inserted between the outer surface of the mask and a retaining component (Figure 21A) and one of a plurality of adjustable holes of the strap may be inserted into a retaining element to adjust the length of the strap (Figure 21B). The remaining portion of the strap may be secured the side structures of the retaining component (Figure 21C).

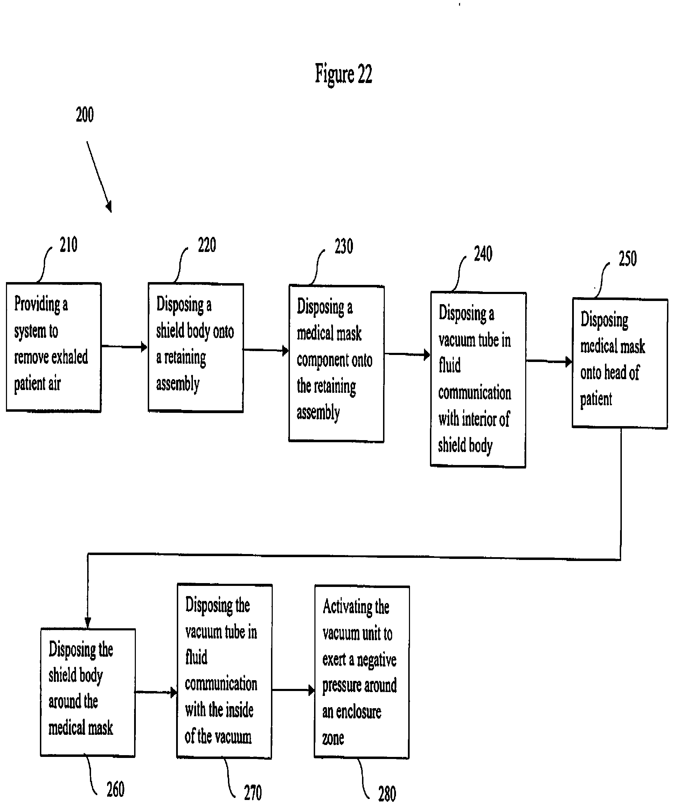

With reference now to at least Figure 22, the present invention is also directed towards a method 200 of removing exhaled air from a patient. As shown at 210, the method 200 comprises providing (i) providing a system 1' as defined herein configured to remove exhaled air from the patient wearing the medical mask. The system 1' may comprise: a vacuum unit 80, a vacuum tube 40; an air filter 70 operatively disposed on an inside of the vacuum tube 40; a vacuum shield assembly 10 comprising a shield body 11 that is disposable onto the patient wearing the medical mask; and a retaining assembly 20 structured to retain the vacuum tube and a component of the medical mask; wherein the vacuum tube 40 is disposed in fluid communication with an inside of the shield body 11 and the vacuum unit 80, and wherein the vacuum tube 40, the shield body 11 and the vacuum unit 80 are collectively disposable into and out of an operative orientation and an inoperative orientation. The method 200 may further comprise: (ii) disposing the shield body 11 onto the retaining assembly 20, which is shown at 220; (iii) disposing the component of the medical mask onto the retaining assembly 200, which is shown at 230; (iv) disposing the vacuum tube 40 in fluid communication with an inside of the shield body 11, which is shown at 240; (v) at least partially disposing the medical mask on the head of the patient, which is shown at 250, and (vi) at least partially disposing the shield body 11 around the medical mask, which is shown at 260. The method 200 may further comprise at least partially disposing the shield body 11 around the medical mask comprises at least partially disposing the shield body 11 around the medical mask and in proximity to the face of the patient creating an enclosure zone 19. As shown at 270, the method 200 may further comprise (vii) disposing the vacuum tube in fluid communication with an inside of the vacuum unit, and as shown at 280 (viii) activating the vacuum unit to exert a negative pressure on the enclosure zone and remove exhaled air from the patient. The method 200 may further comprise (viii) activating the vacuum unit 80 to exert a negative pressure on the inside of the vacuum tube 40 and on the inside of the shield body 11 to remove exhaled air from the patient between the inside of the shield body 11 and the face of the patient.

### II. INTUBATION ASSEMBLY TO PROTECT FROM AIRBORNE ILLNESSES

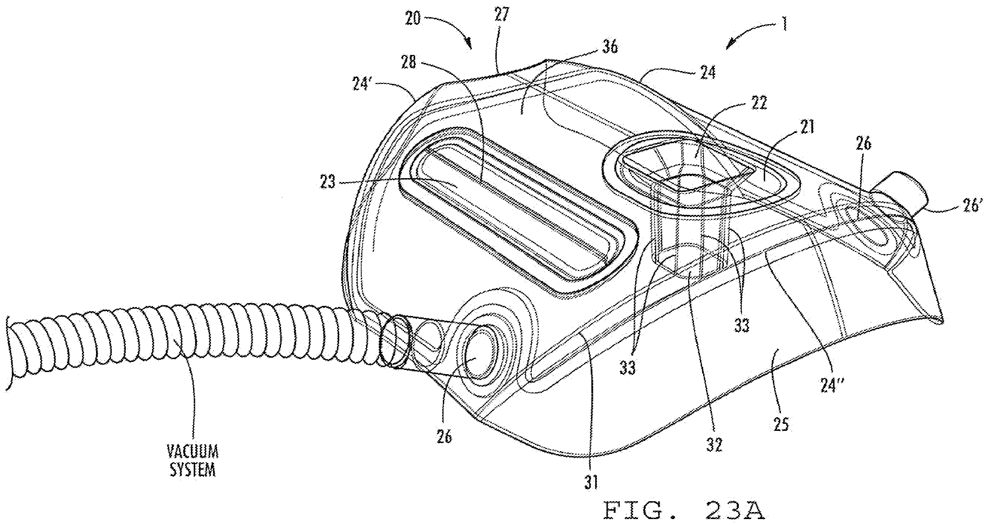

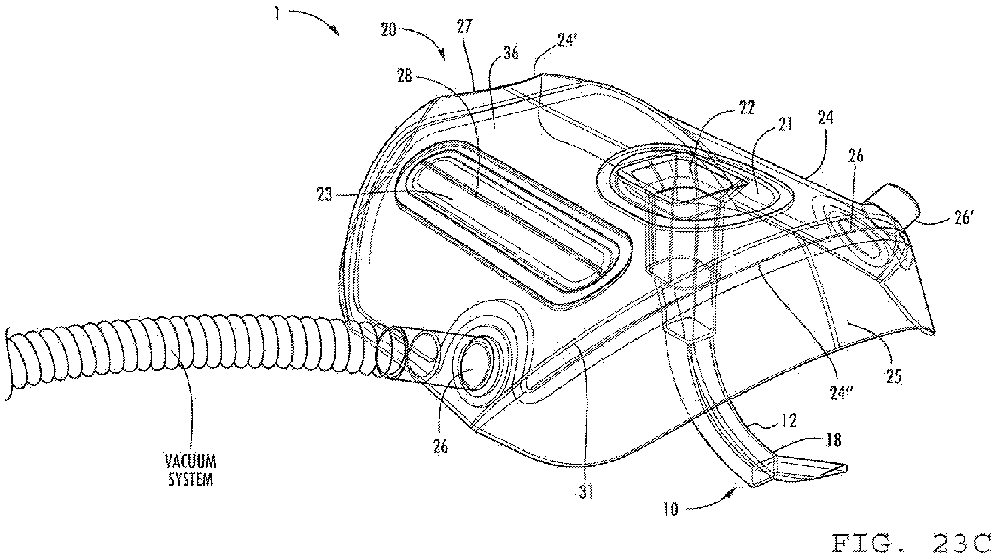

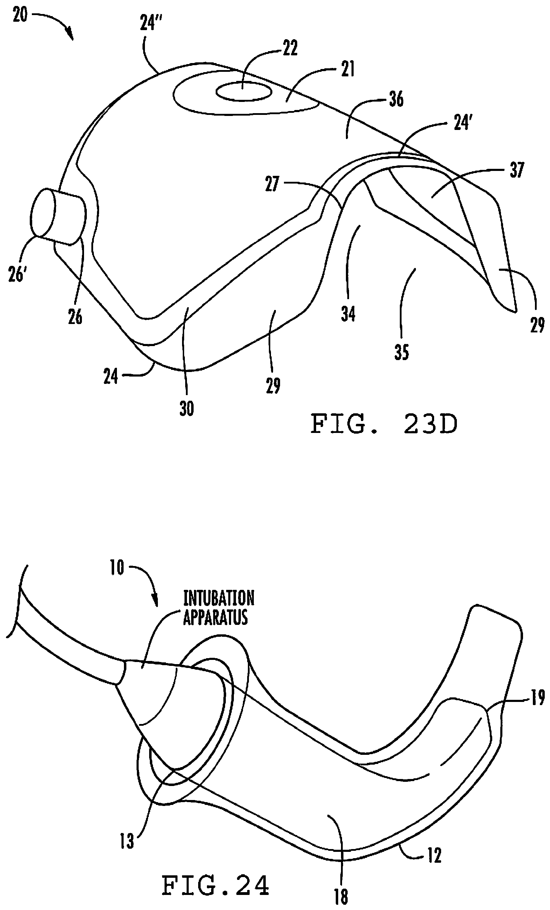

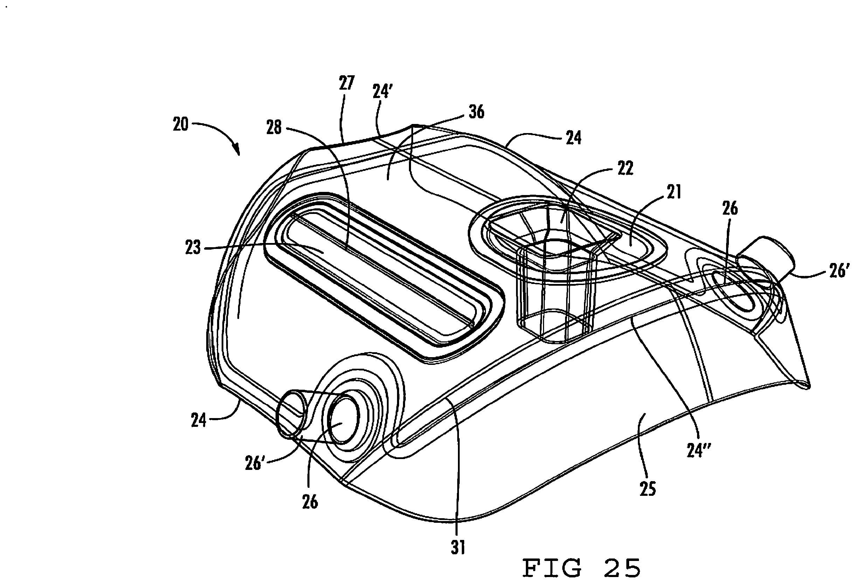

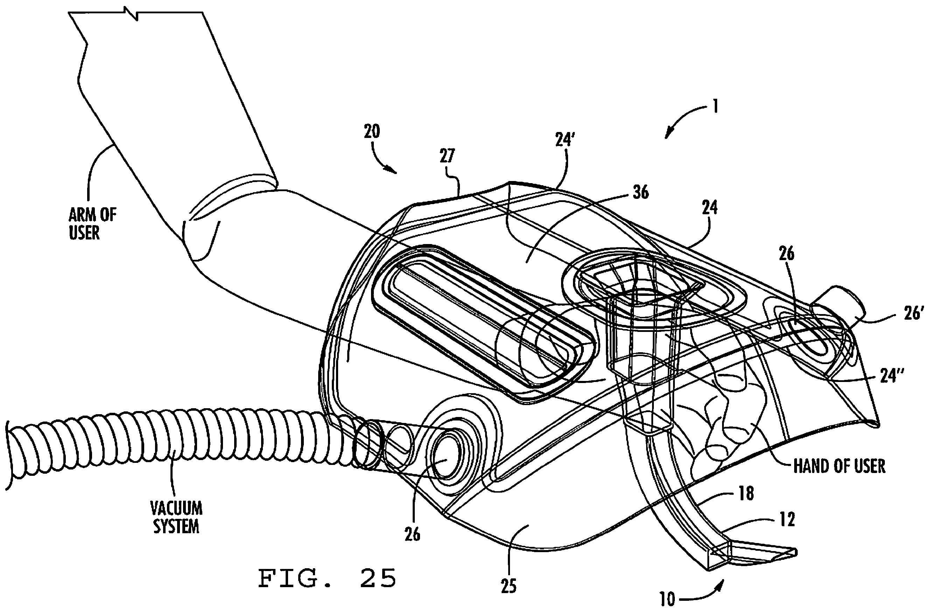

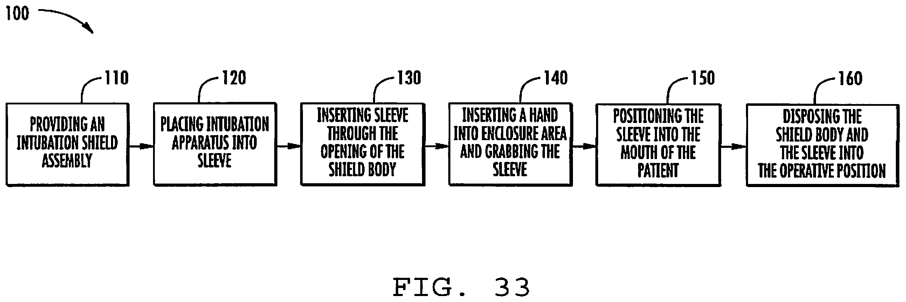

With initial reference to at least Figures 23A-23D, the present invention relates to an intubation assembly, which is indicated at 1. With reference to at least Figure 25, the present invention is also directed to a shield assembly 20. With reference to Figure 33, the present invention is further directed to a method 100 of using the intubation assembly 1. It is within the scope of the present invention that the intubation assembly 1 and/or shield 20, using a negative pressure vacuum, at least partially reduce the risk of contagion of airborne illnesses, including from a patient to a health practitioner, i.e., physician, nurse, assistant, etc. That is, the intubation assembly 1 and/or shield 20 may act as a physical barrier that may at least partially reduce airborne movement of infectious particles, including but not necessarily limited to viruses, e.g., influenza or COVID-19, or bacteria, fungi, etc. The intubation assembly 1 and/or shield 20 may also at least partially reduce the risk of contagion of airborne illnesses from the health practitioner to the patient. The intubation assembly 1 generally comprises an intubation apparatus assembly 10 and a shield assembly 20. The intubation apparatus assembly 10 may be operatively disposed on a shield assembly 20. With reference to at least Figure 24, it is within the scope of the present invention that the intubation assembly 1 and/or shield 20 be used with an intubation apparatus. As used herein, an "intubation apparatus" may include, without limitation, laryngoscopes, endoscopes, bronchoscopes, and other fiberoptic devices. As also used herein, an "intubation apparatus" may also refer to an endoscope assembly, or other related apparatus, as may be used, without limitation, in connection with an upper gastro intestinal (GI) endoscopy (EGD) or similar procedure. Furthermore, it is also contemplated that the inventive intubation assembly 1 be disposable. However, this is not strictly necessary as the inventive intubation assembly 1 may also be disinfected so that it may be used more than once.

As is shown at least in Figure 24, and as mentioned above, the inventive intubation assembly 1 comprises an intubation apparatus assembly 10. The intubation apparatus assembly 10 comprises an intubation apparatus, which may be disposed on a sleeve 12. The sleeve 12 is generally dimensioned to accommodate the size and/or length of the body 18 of the intubation apparatus. It is within the scope of the present invention that the sleeve 12 comprise a material that may create sufficient frictional resistance to engage the intubation apparatus once it is inserted inside of the sleeve 12. The sleeve 12 should also comprise a material that is sufficiently flexible may conform to the geometry of the intubation apparatus. It is also contemplated that the material of the sleeve 12 also permit a manual insertion of the intubation apparatus through a sleeve opening 13.

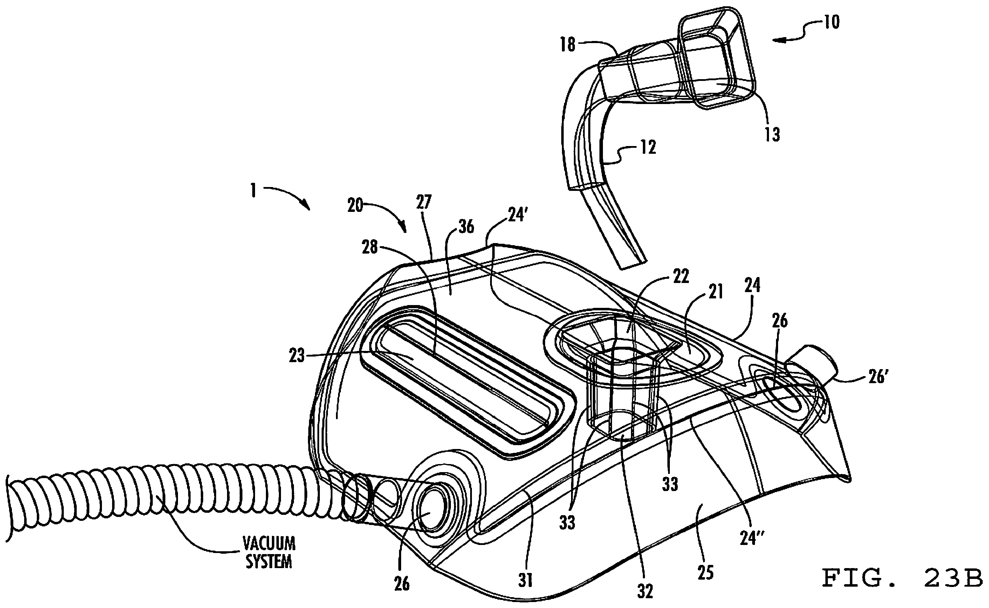

With reference to at least Figures 23B and 24, it is also contemplated that the size and geometry of the intubation apparatus assembly 10, including the intubation apparatus and/or sleeve 12, be configured and dimensioned to permit a health practitioner to hold it with a hand(s) after insertion onto the shield assembly 20, which will be described below. As shown at least in Figure 23B, the sleeve 12 may comprise an elongated and/or curved profile. In the illustrative embodiment of Figure 24, the sleeve 12 may comprise a substantially square opening, including with rounded corners. Alternatively, as shown at least in Figure 24 the sleeve 12 may comprise a substantially circular opening 13 at one of its ends. With reference again to Figure 23B, it is contemplated that the opening 13 of the sleeve 12 be sufficiently large to accommodate insertion of an intubation apparatus. Furthermore, in embodiments comprising a substantially rectangular opening, it is contemplated that the size and configuration of the shield opening 22 be configured and dimensioned accordingly to permit insertion of the sleeve 12 into the shield body 24. Similarly, in embodiments comprising a substantially cylindrical configuration, it is contemplated that the size of the opening 13 of the sleeve 12 comprise a diameter that is at least larger than the diameter of the shield opening 22, which will also be described in more detail below. Furthermore, the sleeve 12 may also comprise a tapered configuration, which may allow the health practitioner to insert it into the mouth, larynx, esophagus, and/or trachea of the patient. As is also appreciated in Figure 24, the laryngoscope may also comprise an audiovisual component 19. For example, the audiovisual component 19 may comprise a camera.

With reference now to at least Figure 25, and as also referenced above, the present invention is directed towards an intubation assembly 1 comprising a shield assembly 20 as well as other embodiments comprising only the shield assembly 20. The shield assembly 20 comprises a body 24, which may comprise a plurality of side segments 29. The shield body 24 may comprise a top surface 36 and a bottom surface 37 as well as proximal end 24' and a distal end 24". The shield body may comprise a variety of shapes and/or configurations, including, without limitation, a substantially arched configuration as is shown throughout the figures. However, this is not necessarily limiting as other shapes and/or configurations are also possible. The shield body 24 may primarily comprise a substantially transparent or translucent material. For example, the body 24, including the side segments 29 may primarily comprise a clear plastic. The material of the body 24 may comprise a rigid clear plastic. The material of the side segments 29 may potentially comprise a flexible material that may allow for further positioning adjustments 29 of the shield body and/or which may at least partially reduce the risk of injury to the patient, as for example, with the flexible material of the top portion 25. The shield assembly 20 may comprise a first transparent component 21, which is disposed substantially around the middle of the body 24. The first transparent component 21 may comprise a substantially transparent or translucent material. Further, the first transparent component 21 may also comprise a shield opening 22 for insertion of the intubation apparatus assembly 10. By way of example, the first transparent component 21 may comprise a clear silicone sheet with an opening disposed thereon. The shield assembly 20 may also comprise a second transparent component 23. The second transparent component may comprise a slot(s) 28 and enabling a fluid communication between the area above the top surface 36 of the shield body 24 and the area below the bottom surface of the shield body 37. It is within the scope of the present invention that the slot(s) 28 remain substantially closed unless the health practitioner selectively opens them, for example to insert an endotracheal tube, which will also be described in more detail later. As an example, the second transparent component 23 may comprise a clear silicone sheet a longitudinally disposed slot(s) 28.

Also with reference to at least the illustrative embodiment shown in Figure 25, the shield assembly 20 of the intubation assembly 1 may comprise a top portion 25. The top portion 25 may be disposed on the distal end 24" of the shield body 24 and may comprise an elongated configuration. The top portion 25 may also comprise a substantially rounded or curved configuration. As shown at least in the illustrative embodiments of Figures 23A-23B and 25, the top portion 25 may be deposed at a downward inclination relative to the shield body 24. The top portion 25 may potentially comprise a flexible material to permit adjustments by the user and/or medical practitioner when positioning the shield body 24 on or around the patient, and/or to at least partially recue the risk of injury to the patient, for example to the neck or chest area of the patient. As such, the top portion 25 as well as the bottom surface 37 of the shield body 24 may substantially define an enclosure are 34, which is shown at least in Figure 23D. The top portion 25 may also comprise a substantially soft or malleable material. For example, the top portion may comprise a soft silicone material. Furthermore, the top portion may comprise at least one vacuum opening 26. The vacuum opening 26 may be disposed in fluid communication with a vacuum connecting portion 26', which itself may be disposed in fluid communication with a vacuum tube. As such, a negative pressure may be transferred from the vacuum tube and/or system and exerted on the enclosure area 34. As shown in the illustrative embodiment of Figure 25, two vacuum openings 26 may be provided. Further, a cap may be provided to cover one or both vacuum openings 26 and/or vacuum connecting portions 26". As an example, the vacuum openings 26 may comprise hose ports. The vacuum openings 26 may be operatively disposed with a vacuum system to provide negative pressure. As used herein, a "vacuum system" may refer to one or more components associated with vacuum equipment, including a vacuum tube or conduit that may exert a negative pressure, and/or other associated components, including, without limitation a vacuum machine and/or filtering apparatus. As such, and with reference to at least Figures 23A-23D and 25, one or more vacuum tubes may be operatively disposed on the vacuum openings 26 to enable a negative pressure on the side of the shield body 24 disposed against the face of the patient. As such, given the operative arrangement enabled by the geometry of the shield body 24 and vacuum openings 26, the resulting negative pressure should provide for an at least partially increased removal of exhaled infectious particles.

As is also shown at least in Figure 23D, the shield assembly 20 may comprise a curvature 27. The curvature 27 may be substantially defined by the geometry of the ends of the side segments 29, which may for example comprise an elliptical configuration. The side segments 29 may be disposed on the proximal end 24' of the shield body 24, and further, may be disposed in a spaced apart relation to one another. As shown at least in Figure 23D, the spaced apart relation between the side segments 29 may at least partially define an aperture 35. As such, the curvature 27 of the body 24 is advantageous to accommodate the arm of a health practitioner when holding the intubation apparatus assembly 10. Further, it is within the scope of the present invention that the curvature 27, the side segments 29, and/or the shield body 24, be configured and dimensioned to define an aperture 35 of a geometry and/or a sufficient dimension that may allow a user or practitioner to place his/her hand through the aperture 35 and into the enclosure area 34. As such, the user or practitioner may grab a sleeve 12 that has been inserted into the shield body 24.

With reference now to Figures 23B and 26, and as mentioned above, it is within the scope of the present invention that the intubation apparatus assembly 10 be inserted into the sleeve opening 13 of the shield body 24. As such, a health practitioner may grab and/or position the intubation apparatus assembly 10 on an intended area of the body of the patient, e.g., the mouth, larynx, esophagus, and/or trachea. Because the diameter of the substantially circular configuration 13 of the sleeve 12 should be at least larger than the diameter of the shield opening 22, the sleeve 12 should be retained around its end on the first transparent component 22. However, the sleeve 12 should protrude on an opposite side of the shield body 24, i.e., the side facing the patient. As is perhaps best shown in Figure 26, the sleeve 12 should remain movable with respect to the shield body 24 after insertion. However, the sleeve 12 should substantially pass through shield opening 22.

As is perhaps best shown in the illustrative embodiment of Figure 25, the shield body 24 may comprise a sleeve retainer 32 disposed on the bottom surface 37. The sleeve retainer 32 is intended to act as a guide and/or support to the sleeve 12 once it has been inserted into the shield body 24. Accordingly the sleeve retainer 32 may be a channel or conduit which may extend below the shield opening 22 in a direction that is substantially perpendicular to the bottom surface 37. The sleeve retainer 32 should comprise an opening that corresponds to the shape of the shield opening 22 and/or sleeve 12, e.g., substantially square or substantially circular. The sleeve retainer 32 should allow for movement and/or adjustment of an inserted sleeve 12. For example, sleeve retainer 32 may be provided with four adjacently disposed walls to form a substantially square configuration extending away from the bottom surface 37. The sleeve retainer 32 may be provided with a recessed wall(s). That is, at least one of the adjacently disposed walls of the sleeve retainer 32 may comprise a lesser length that the other three to at least partially allow for movement and/or adjustment of the sleeve 12 in at least one direction. Additionally, an expandable component 33 may be provided along one or more of the walls to further allow for further movement and/or adjustment of the sleeve 12 in at least one direction, and in some embodiments in several directions. The expandable components 33 may comprise a corrugation or groove within the walls of the sleeve retainer 32, which may at least partially allow for such further movement and/or adjustment of the sleeve 12 once it has been inserted.

With reference now to at least Figures 23A-23D and 25, the shield assembly 20 may be provided with a reinforcement component 30 and/or an overlapping portion 31. A reinforcement component 30 and/or overlapping portion 31 may be provided to at least partially increase the stability of the shield body 24 and/or at least partially reduce bending of the shield body 24. As may be appreciated at least in Figure 23D, the reinforcement component 30 may be disposed or otherwise formed on the top surface 36 of the shield body 24 around is proximal end 24'. The reinforcement component 30 may extend substantially along the width of the top surface 36 of the shield body. The reinforcement component 30 may comprise an elongated configuration and/or may follow or otherwise correspond to the profile of the curvature 27. The reinforcement component 30 may also be provided at an offset with respect to the curvature 27 and/or side segments 29. Conversely, and as may be appreciated at least in Figures 23A-23C and 25, the overlapping portion may be disposed or otherwise formed on the top surface 36 of the shield body 24 around its distal end 24". The overlapping portion 31 may be formed by an overlay or otherwise intersection between the shield body 24 and the top portion 25.

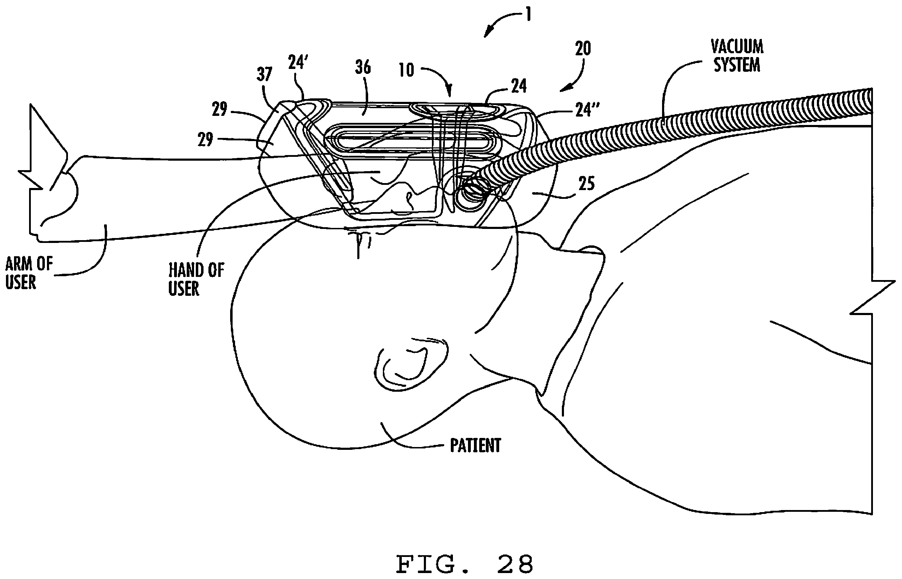

With reference now to Figures 26-30, features of the present invention comprise disposing the inventive intubation assembly 1 and/or shield assembly 20 into and out of an operative position and an inoperative position. As used herein, the "inoperative position" of the inventive intubation assembly 1 and/or shield assembly 20 refers the non-operation and/or storage of the various components of the intubation assembly 1 and/or shield assembly. The "inoperative position" may also refer to a position of the intubation assembly 1 and/or shield assembly 20 that does not involve placing the sleeve 12 into the shield body 24 or that does not involve dispose the shield assembly 20 in proximity to the face and/or head of the patient. Furthermore, the "inoperative position" may also refer to the non-operation of a vacuum system connected to the shield assembly 20 and/or shield body 24. With reference to Figure 28, and as used herein, the "operative position" of the intubation assembly 1 and/or shield assembly generally comprises the intubation apparatus assembly 10, sleeve 12 and/or intubation apparatus, operatively disposed on the shield 24, i.e., inserted through the shield opening 22. The "operative position" also refers to the intubation apparatus assembly 10, sleeve 12 and/or intubation apparatus disposed the mouth, larynx, esophagus, and/or trachea of the patient, which for simplicity may be collectively referred to as the mouth of the patient. The "operative position" may also refer to the shield body 24 disposed in proximity to the patient, and/or the bottom surface 37 facing the patient. Furthermore, the "operative position" may comprise the vacuum system connected to the vacuum opening 26 and/or vacuum connecting portion 26', and being disposed in an operational setting, i.e., exerting a negative pressure, such that a negative pressure is transferred to the enclosure area 34 and/or the area surrounding the patient. As such, at least partially exhaled air may be extracted from the area surrounding the patient, while the user or practitioner performs an intubation procedure.

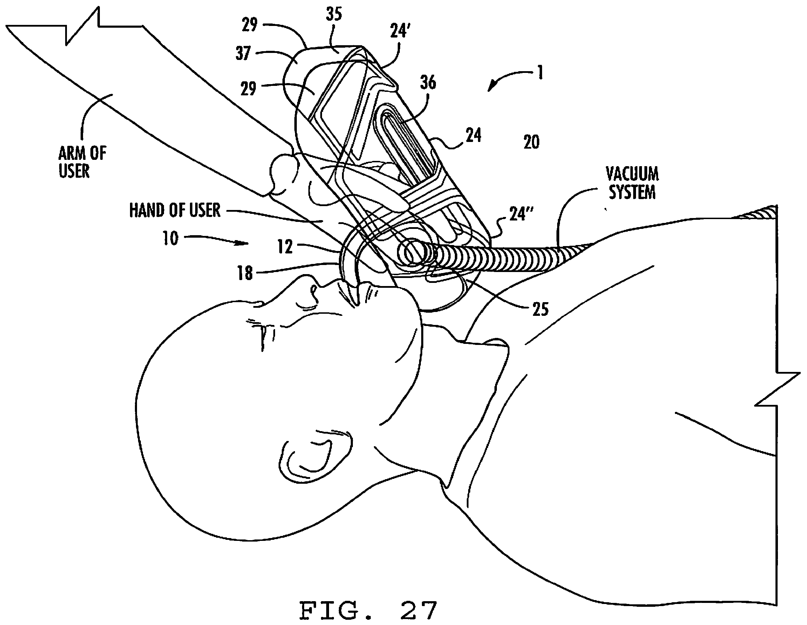

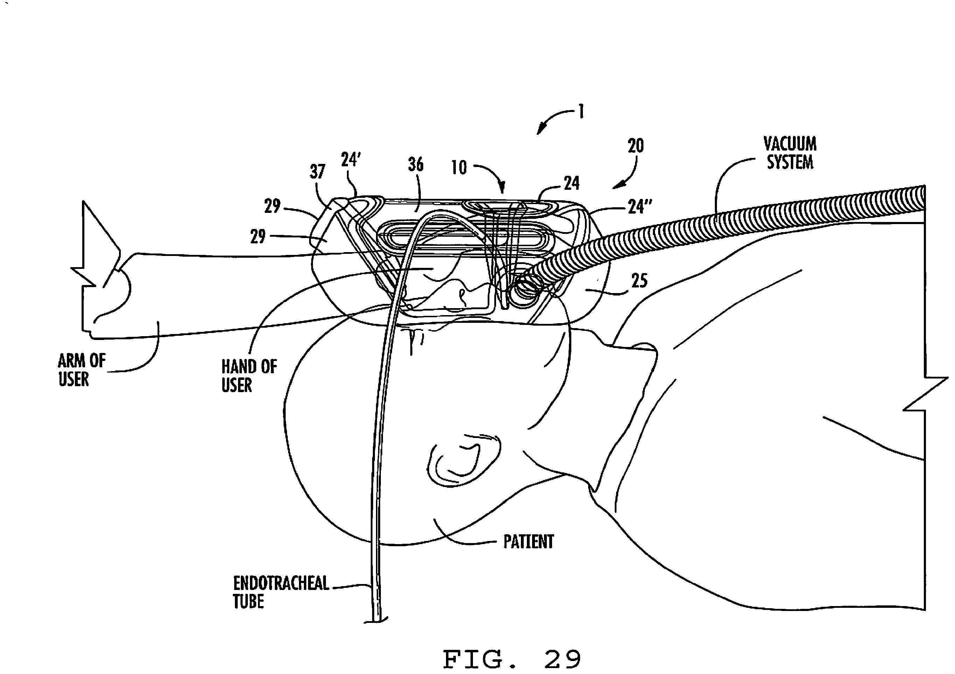

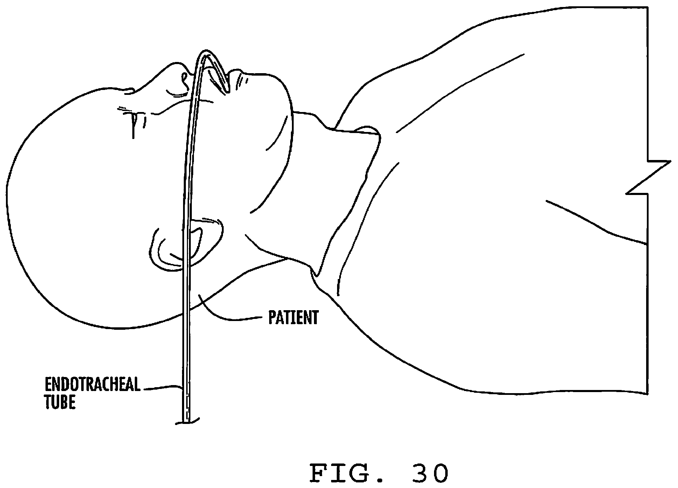

As shown in Figure 27, the shield assembly 20, with the intubation apparatus assembly 10 and/or sleeve 12 inserted, may be positioned at a slight inclination with respect to the body of the patient. This at least partially facilitates an initial insertion of the sleeve 12 into the mouth of the patient. Here, the top portion 25 of the shield assembly 20 may come into contact with the neck of the patient. Accordingly, it is advantageous to provide a top portion 25 with a substantially soft material, e.g., soft silicone, to at least partially reduce the risk of physically injury, including around the neck of the patient. Once the sleeve 12 is initially inserted into the mouth of the patient, the shield assembly 20 may be manually moved from the position represented in Figure 27 to the position represented in Figure 28. During this process, the sleeve 12 may be inserted deeper into the mouth, and into the larynx, esophagus, and/or trachea of the patient. As shown in Figure 29, once the shield assembly 20 and/or the intubation apparatus assembly 10 are disposed in an operative position, i.e., the position as represented in Figure 28, an endotracheal tube may be passed through a slot 28 from an outside of the shield body 24 to the opposite side facing the patient. After being inserted through the at least one of the slots 28, the endotracheal tube may be placed on mouth of the patient. Thereafter, the endotracheal tube may be selectively positioned on an intended area of the patient, for example, an intended location of the larynx and/or trachea. At this stage, the audiovisual component 19 may assist the health practitioner in positioning the endotracheal tube on the intended area of the patient. As shown in Figure 30, after the endotracheal tube has been inserted and/or positioned on an intended area of the patient, the shield assembly 20 and intubation apparatus assembly 10 may be removed.

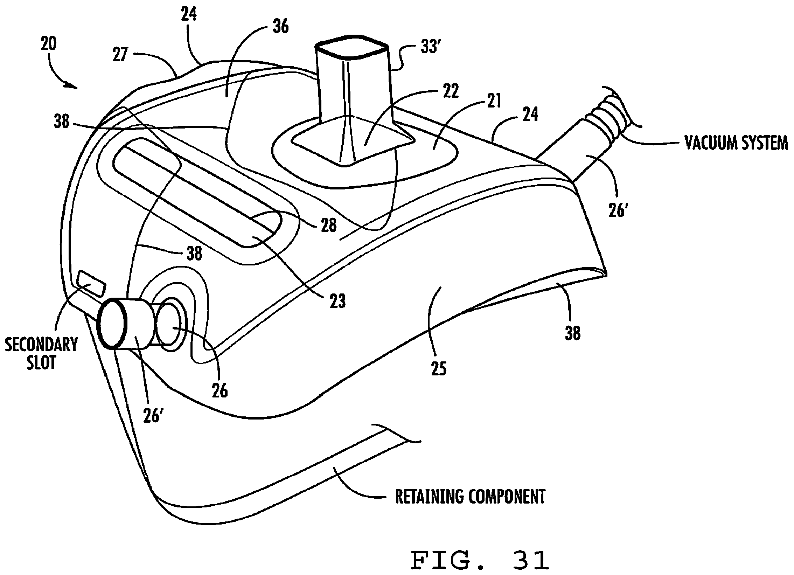

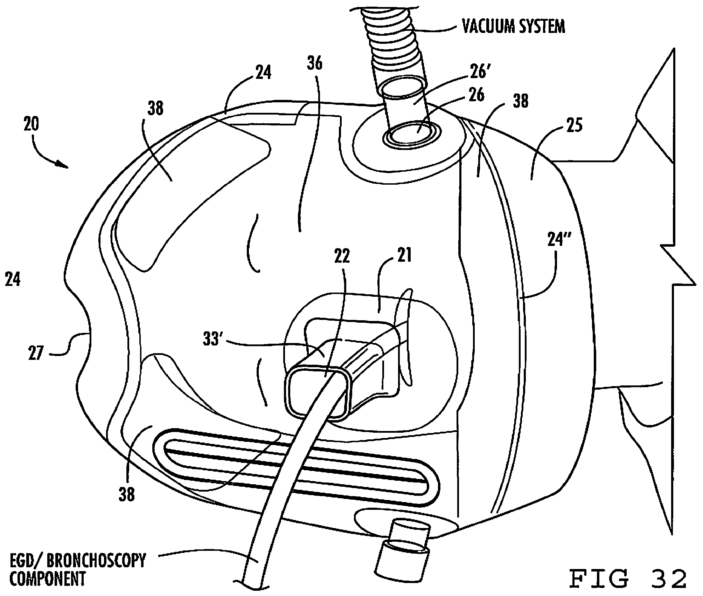

With reference to Figures 31-32, additional features of the present invention comprise providing a shield assembly 20 and/or an intubation assembly 1 with an offset structure 38. In some applications, it may be of interest that the shield body 24 be connected to the head of the patient, to at least partially limit movement of the shield 24 with respect to the body of the patient. For example, during an upper GI endoscopy (EGD) and/or a bronchoscopy, a retaining component, i.e., adjustable straps, ties, bands, etc., may be provided to dispose the shield body 24 around the head of the patient. The retaining component should at least partially reduce movement of the shield body 24 with respect to the body of the patient. The retaining component may be connected to the shield body around a secondary slot or opening configured and dimensioned for the size of the retaining component. For such applications, e.g., an upper GI endoscopy (EGD), a bronchoscopy and/or a related procedure, a shield body 20 may be provided with an offset structure 38, which is primarily intended to provide for a separation between the shield body 24 and the body of the patient, inducing the head, forehead, face, neck, upper portion of the chest, and/or shoulders, etc. It is within the scope of the present invention that such separation between the body and the offset structure 38 may be beneficial to at least partially protect the areas of the patient that may otherwise be in direct contact with the shield body 24, e.g., head, forehead, face, neck, upper portion of the chest, and/or shoulders. Accordingly, an offset structure 38 may be provided around the bottom surface 37 of the shield body 24.

As seen in Figures 31-32, the offset structure 38 may be provided around the bottom surface 37 of the shield body 24, including around the top portion 25 and/or the side segments 29. By way of example only, the offset structure 38 may comprise a foam pillow liner or other related material, e.g., a foam pad, such that it may serve to elevate or otherwise raise the position of the shield body 24 with respect to the body of the patient. As such, once the shield body 24 is disposed on the head of the patient, the intubation apparatus, e.g., bronchoscope and/or endoscope, may be inserted through the shield opening 22. In such embodiments, it is further contemplated that the vacuum opening(s) 26 and/or vacuum connecting portion(s) 26 also operate as described herein, to at least partially remove exhaled air from the patient with an operatively connected vacuum system. In such embodiments, and in addition to, or in lieu of, the offset structure(s) 38, a retainer 33' may be provided. The retainer 33' may be oriented outwards, i.e., away from the top surface 36, instead of inwards, i.e., away from the bottom surface 37 and/or towards the patient. A retainer 33' may be provided to further assist the user or practitioner to insert the intubation apparatus, e.g., EGD apparatus, bronchoscope and/or other related apparatus may be inserted through a retainer 33' disposed around the shield opening 22. As with the sleeve retainer 33, the retainer 33' is intended to function as a guide for the intubation apparatus.

With reference now to Figure 33, the present invention is further directed to a method 100 of using the inventive intubation assembly 1. As shown at 110, the method 100 initially comprises providing an intubation assembly 1 comprising an intubation apparatus assembly 10 and a shield assembly 20 as described herein. As shown at 120, the method 100 further comprises placing an intubation apparatus into a sleeve 12 of the intubation apparatus assembly 10 of the inventive intubation assembly 1. As shown at 130, the method 100 further comprises inserting the sleeve 12 through the opening 22 of the shield body 24. As shown at 140, the method 100 further comprises inserting a hand of a user into the enclosure area 34 and grabbing the sleeve 12. As shown at 150, the method 100 further comprises positioning the sleeve 12 into the mouth of the patient. It should be understood that once the sleeve 12 is positioned into the mouth of the patient, that the shield body 24 will at least partially surround the face and/or head of the patient, and that the bottom surface 37 of the shield body 24 should face the patient. As shown at 160 the method 100 further comprises disposing the shield 24 assembly and the intubation apparatus assembly into the operative position.

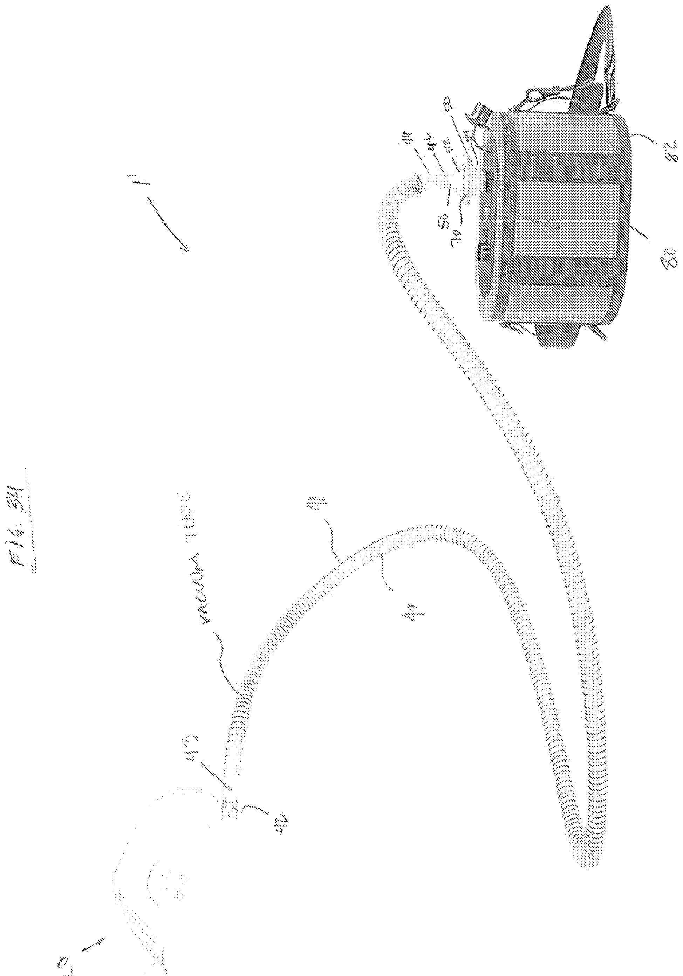

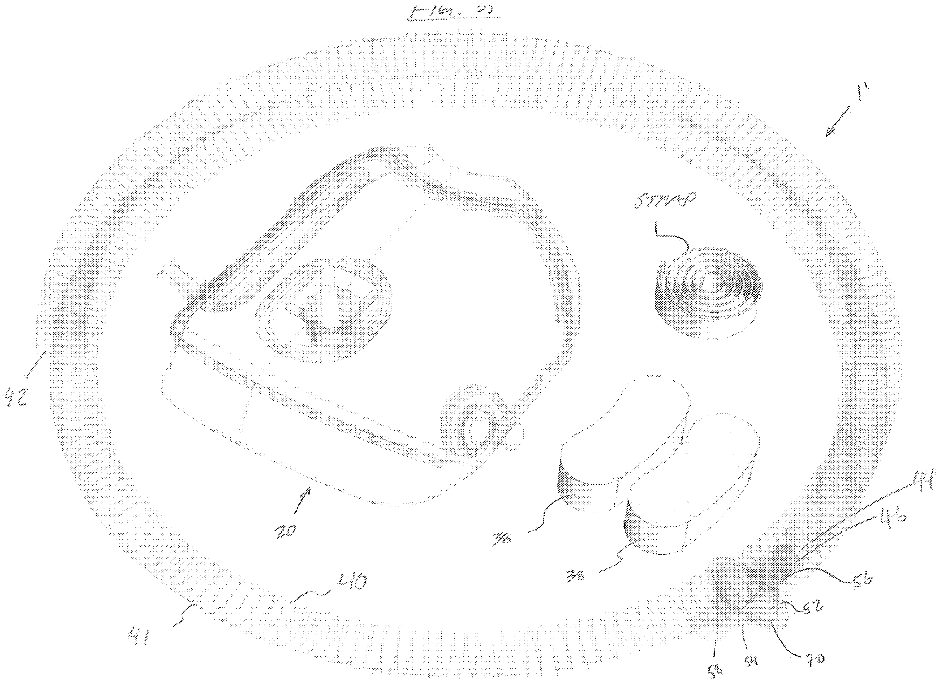

With reference to Figures 34-40, further embodiments of the present invention relate to a system 1' configured to remove exhaled air from a patient. Generally, the system 1' according to the present invention is configured to remove exhaled air from a patient by placing a shield body 24 around the upper body area of the patient, i.e., neck and/or face area. With reference to at least Figures 34 and 35, the system 1' generally comprises a shield assembly 20 as defined herein, including a shield body 24 and/or 62, a vacuum tube 40 and a vacuum unit 80. As used herein, a vacuum unit 80 refers to a vacuum device, which may be motor operated, and which may be disposed in fluid communication with a vacuum tube 40, i.e., a hose, or other flexible or expandable hollow elongated component, and which may exert a negative pressure. It is contemplated that a shield body 24 of the shield assembly 20 also be disposed in fluid communication with the interior of the vacuum tube 40. The negative pressure of the vacuum unit 80 should be transferred through the vacuum tube 40, and to an interior face of the shield body 24, i.e., the area defined below bottom surface 37, the side of the shield body 24 that faces the patient and/or the enclosure area 34. As such, the shield body 24 essentially acts as a vacuuming device that is capable of at least partially removing exhaled air around the face of the patient. As used herein, the space defined by the interior face of the shield body 24 as well as the face of the patient, including when wearing the medical mask, is defined as an enclosure area 34.

With reference to at least Figure 35, the vacuum tube 40 may comprise a body 41 of a flexible, or elastic material that may at least partially bend, twist, move, or otherwise conform to geometric constraints. The vacuum tube 40 may comprise a proximal end 44 that connects to the vacuum unit 80 around connecting end 36, and a distal end 42 that connects to the shield body 24 around a connecting end 46. As is also shown at least in Figure 35, the shield body 24 may also comprise a plurality of offset structures 38 which may assist in positioning the shield body 24 on the head of the patient.

With reference to at least Figure 34, a filter case assembly 50 may be disposed or integrally formed on the vacuum tube 40 around the proximal end 44. The filter case assembly 50 may comprise a top segment 52 and a bottom segment 54 which may be operatively connected to one another. In other words, the top segment 52 and bottom segment 54 may form a mating engagement with one another in a secured position, i.e., once a filter 70 has been placed between them. It is contemplated that the mating engagement between top segment 52 and bottom segment 54 not be a permanent mating engagement such that the top segment 52 and bottom segment 54 may removably connected to another to insert and/or remove a filter in the area where they engage. The diameters of the top segment 52 and/or bottom segment 54 should correspond to one another and should be configured and dimensioned to accommodate the diameter and/or size of a filter 70, which may comprise an air filter such as an ultra-low particulate air (ULPA) filter. The filter 70 may also comprise a high efficiency particulate air (HEPA) filter. As such, the diameters of the top segment 52 and/or bottom segment 54 may be larger than the diameter of the vacuum tube 40, but this is not strictly necessary. When the top segment 52 and the bottom segment 54 are forming a mating engaged, this should restrict placement or otherwise movement of a filter 70 disposed therein. Furthermore, the top segment 52 and/or upper segment 54 may be provided with conical or semi-conical shapes. This may be done to at least partially facilitate airflow through the vacuum tube 40 and into the vacuum unit 80 and/or to otherwise at least partially reduce the likelihood of a bottleneck effect around the area where the filter 70 is disposed. Consequently, air captured around a distal end 42 of the vacuum tube will pass through the filter 70 before entering the vacuum unit 80, at least partially reducing contaminants and/or other infectious particles.

With reference to at least Figure 34, and as mentioned above, the system 1' according to the present invention comprises a vacuum unit 80. The vacuum unit 80 should provide for a portable solution of creating a negative vacuum pressure, at least around its first opening 84 where the vacuum tube 40 will be connected. The vacuum unit 80 may comprise a housing 82 with a first opening 84 and a second opening 86. The first opening 84 is generally configured for attachment of the vacuum tube 40, for example, around a proximal end 44 thereof. The second opening 86 is generally configured for captured air to exit outside of the housing 82. That is, air collected form the enclosure area 34 that passes through the first opening 84 and into the interior of the housing 82, should be able to exit outside of the housing 82 through the second opening 86. Alternatively, other means of air escape may be provided on the housing, and may include slots or vents, including disposed on the sides. In addition, the interior of the housing 82, which is generally a chamber, may be provided with other filtering means to further remove contaminants and/or infections particles form the air captured around the enclosure area 34. Also, a top cover of the housing may be removable from the rest of the housing 82 to access any components thereof, which may include a battery-operated vacuum with a motor, additional filtering components, etc. By way of example only, a battery-operated vacuum motor of 110V or similar, may be provided on an inside of the housing 82. In at least one embodiment, a battery operated vacuum motor of 110V may be provided on an inside of the housing 82.

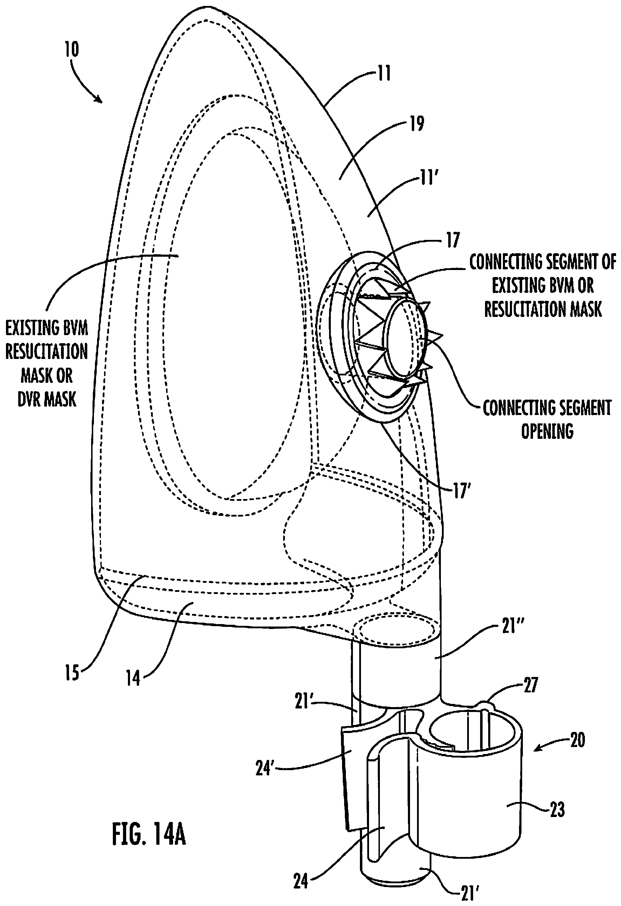

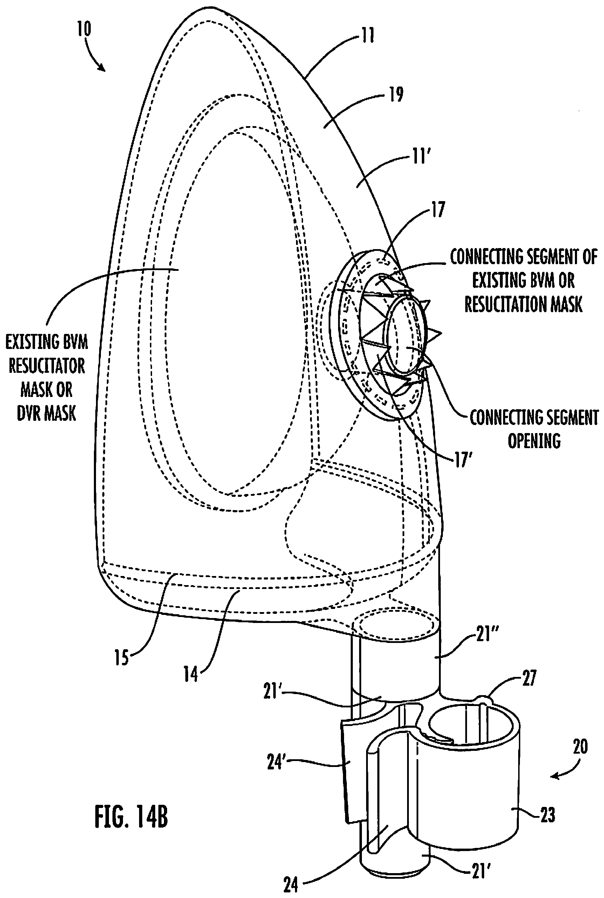

Specific to the system 1' according to the present invention, the vacuum unit 80, vacuum tube 40 and shield body 24 may be collectively disposable into and out of an operative orientation and an inoperative orientation. The operative orientation comprises the vacuum unit 80 activated and exerting a negative pressure on an inside of the shield body 24, i.e., around the enclosure area 34, to at least partially remove exhaled patient air. The inoperative operation comprises periods of non-operation of the system 1', including when the vacuum unit 80 is inactive. It is contemplated that the shield body 24 may be disposed into and out of an operative position, as shown at least in Figures 38B and 14E. The operative position generally comprises an intubation apparatus disposed on the shield opening 22 and into the mouth of the patient. The operative positon may also comprise the interior of the shield body 24, i.e., the bottom surface 37, facing the patient. Alternatively, and with reference again to at least Figure 40, the operative position may comprise an intubation apparatus disposed into the opening 68 of a shield body 62 and into the trachea of the patient through a neck opening, with the interior or inner surface 63' of the shield body 63 disposed against the neck of the patient. In the operative orientation, the shield body is disposed into the operative position and the vacuum unit 80 is activated to exert a negative pressure on an inside of the shield body, or otherwise the enclosure area 34, at least partially removing exhaled air from the patient.

With reference to at least Figures 38-40, in an alternative embodiment, the system 1' according to the present invention may be implemented with a trachea shield assembly 60 comprising a shield body 62 that is placed around the neck area of a patient to access the trachea. In such embodiments of the inventive system 1', it is contemplated that a trachea shield assembly 60 may be provided on order to perform an intubation or related procedure around the trachea area of the patient, e.g. a tracheostomy or a bronchoscopy. The trachea shield assembly 60 primarily comprises a shield body 62 a strap or other component used to retain the shield body 62 around the neck of the patient. As seen at least in Figures 39-40, the shield body 62 may comprise an upper end 67 and a lower end 61 designed to expand above and below an opening of the neck (shown in Figure 38). The shield body 62 may comprise an opening 68, with an optional cap or other sealing mechanism. The location of the opening 68 should correspond to the approximate location of the neck opening. As such, the opening 68 may permit insertion of intubation components directly to the trachea of the patient without requiring insertion through the mouth of the patient. It is contemplated that at least one vacuum opening 66' be provided on either side of the opening 68 to allow for other components to be attached to the shield body 62 around an outer surface 13 thereof. An inner surface 13' of the shield body 62 should be disposed against the neck area of the patient. Such other components that may be attached to the shield body 62 may comprise a vacuum tube 40, which may be connected around a connecting segment 66'. As shown in Figure 40, an oxygen supply tube may also be connected to the connecting segment 16'. As such, the components connected through the openings 66, i.e., oxygen supply tube and/or vacuum tube, should be in disposed in fluid communication with an interior or inner surface 13' of the shield body 62. Furthermore, a safety inhalation valve and/or a safety flutter valve 64 may also be disposed on the shield body 62 as an emergency feature in the event of patient asphyxiation or an oxygen feed failure or other failure.

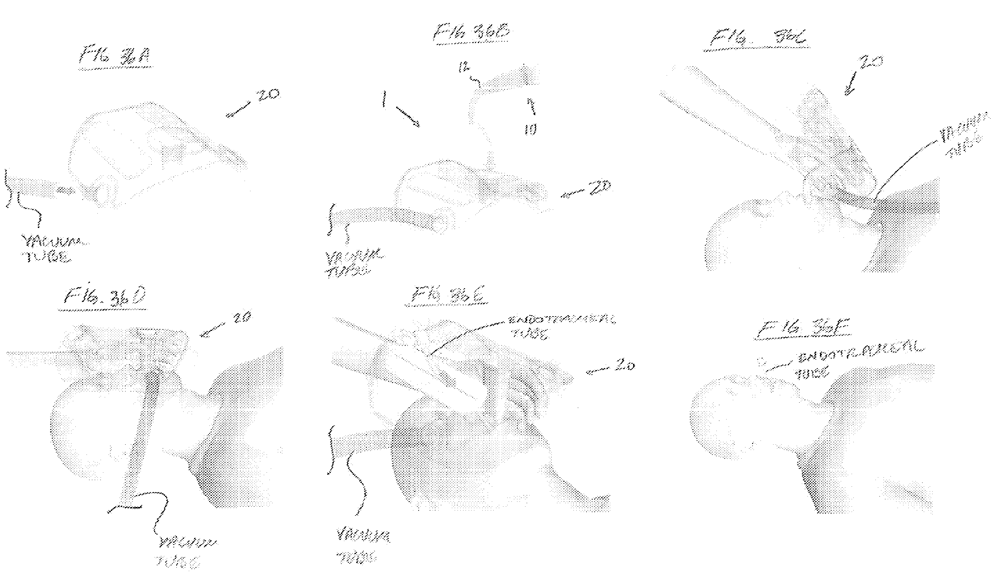

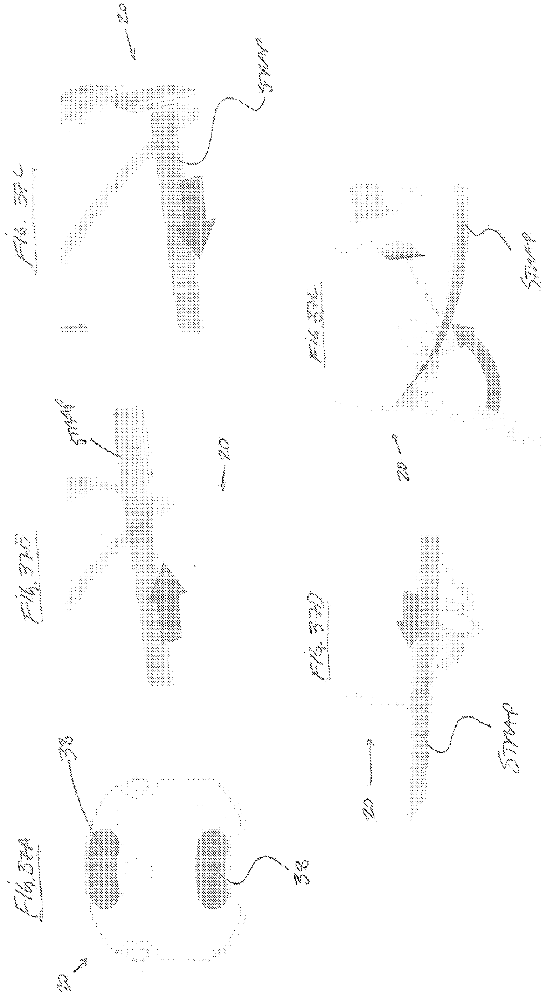

With reference now to at least Figures 36A-36D , and as mentioned above, various components of the innovative system 1' may be disposed into an operative position as shown in at least Figure 36E and 38B. The shield body 24 and/or 62 may be connected to the vacuum tube and/or oxygen supply tube (e.g., Figure 36A). Thereafter, a sleeve 12 or intubation apparatus may be inserted through opening 26 (e.g., Figure 36B). Thereafter, the shield body 24 and/or 62 may be position on the patient (e.g., Figures 36C-36D). There after an endotracheal tube may be inserted through a slot 28 and/or opening 68 and into the mouth and/or trachea of the patient (e.g., Figure 36E). Thereafter, the shield body 24 may be removed once the patient is intubated (e.g., Figure 36F). As shown in Figures 37A-38B and 17, in some embodiments the shield body 25 and/or 62 may be provided with a strap. As shown in Figures 37A-E, the strap may be inserted into a slot(s) of the shield body 24, which may be configured and dimensioned for insertion of the strap. Thereafter, the strap may adjusted to securely retain the shield body 24 onto the head of the patient. A leading end(s) of the strap may be connected to other portions of the same strap, for example, with a hook and loop connection or other connection mechanism.

With reference now to at least Figure 41, the present invention is also directed towards a method 200 of removing exhaled air from a patient. As shown at 210, the method 200 comprises providing (i) providing a system 1' as defined herein configured to remove exhaled air from the patient wearing the medical mask. The system 1' may comprise: a vacuum unit 80, a vacuum tube 40; an air filter 70 operatively disposed on an inside of the vacuum tube 40; a vacuum shield assembly 20 comprising a shield body 24 that is configured and dimensioned for insertion of an intubation apparatus and at least one vacuum opening 26 disposed on the shield body 24; wherein the vacuum tube 40 is disposed in fluid communication with an inside of the shield body 24 and the vacuum unit 80, and wherein the vacuum tube 40, the shield body 24 and the vacuum unit 80 are collectively disposable into and out of an operative orientation and an inoperative orientation. The method 200 may further comprise: (ii) attaching the vacuum tube to the at least one vacuum opening, which is shown at 220; (iii) using the vacuum unit to exert a negative pressure on an inside of the shield body, which is shown at 230; (iv) inserting the intubation apparatus into the mouth of the patient, which is shown at 240; (v) inserting a endotracheal tube through the shield body and positioning the endotracheal tube on the trachea of the patient, which is shown at 250; and (vi) removing the shield body from the patient, which is shown at 260. The method 200 may further comprise inserting the intubation apparatus into the mouth of the patient when the shield body 24 is adjacently disposed to the face of the patient and using the vacuum unit 80 to exert a negative pressure on the enclosure area 34 or otherwise between the shield body 24 and the face of the patient.

Since many modifications, variations and changes in detail can be made to the described preferred embodiment of the invention, it is intended that all matters in the foregoing description and shown in the accompanying drawings be interpreted as illustrative and not in a limiting sense. Thus, the scope of the invention should be determined by the appended claims and their legal equivalents.

## Claims

1. A vacuum shield assembly disposable onto a patient wearing a medical mask and configured to remove exhaled air from the patient, said vacuum shield assembly (10) comprising:
a shield body (11) comprising a concave configuration, said shield body (11) further comprising an edge (12) defining a substantially flat side profile of said shield body (11);
a lower segment (15) disposed on a lower perimeter of said shield body (11), said lower segment (15) comprising a substantially curved configuration and a first opening (13);
a retaining assembly (20) structured to retain an oxygen supply tube or a nebulizer unit of the medical mask; said retaining assembly (20) comprising:
a retaining component (21) configured and dimensioned to retain a connecting portion (18) of said shield body (11), said connecting portion (18) being disposed in fluid communication with said first opening (13) of said shield body (11); and
a retaining frame (23) comprising a semi-cylindrical configuration and connected to said retaining component (21), said retaining frame (23) being configured to retain the oxygen supply tube or a nebulizer unit of the medical mask;
**characterized in that**:
the retaining assembly (20) is further structured to retain a vacuum tube (40);
a lower section (21') of said retaining component (21) is configured and dimensioned for attachment of the vacuum tube (40);
said lower segment (15) is configured to exert a negative pressure on an inside of said shield body (11); and
said shield body (11) and said retaining assembly (20) are collectively disposable into and out of an operative position and an inoperative position; said operative position comprising said shield body (11) adjacently disposed to and facing the medical mask; said operative position further comprising, the vacuum tube (40) exerting a negative pressure on an inside of said shield body (11) at least partially removing exhaled air from the patient between said shield body (11) and the medical mask.

2. The vacuum shield assembly as recited in claim 1 wherein said retaining assembly (20) comprises a first closing mechanism (25) and a second closing mechanism (26) cooperatively configured to adjust an inner area of the retaining component (21); each one of said first closing mechanism (25) and said second closing mechanism (26) disposed on said retaining frame (23).

3. The vacuum shield assembly as recited in claim 2 wherein said first closing mechanism (25) comprises a plurality of serrations (25'); and said second closing mechanism (26) comprises a snap mechanism operatively configured and dimensioned with said plurality of serrations (25') to adjust a sleeve size of said retaining frame (23).

4. The vacuum shield assembly as recited in claim 1 further comprising at least one retaining segment (28) disposed on said retaining frame (23); said at least one retaining segment (28) configured to at least partially retain the nebulizer unit, and wherein said at least one retaining segment (28) comprises a latch (29) disposed on an upper end thereof; said latch (29) structured to at least partially reduce movement of the nebulizer unit in a vertical direction.

5. The vacuum shield assembly as recited in claim 1 wherein said retaining component (21) comprises an upper section (21") configured and dimensioned to retain said connecting portion (18) of said shield body (11).

6. The vacuum shield assembly as recited in claim 1 wherein said lower segment (15) is disposed below said lower perimeter of said shield body (11); said shield body (11) further comprises an upper segment disposed above said lower segment (15); said upper segment comprising an outer surface (11'), an inner surface (11") and a second opening (16) disposed substantially on a middle section of said upper segment and above said first opening (13); said second opening (16) configured and dimensioned to receive a connecting segment of the medical mask, the medical mask being a bag-valve-mask, BVM, resuscitator mask or a demand-valve resuscitator, DVR, mask.

7. The vacuum shield assembly as recited in claim 6 further comprising a vacuum attachment (17) substantially disposed around said second opening (16), wherein said vacuum attachment (17) is configured to substantially form a seal between said outer surface (11') and said inner surface (11") around said second opening (16) when said connecting segment of the BVM resuscitator mask or the DVR mask is inserted on said second opening (16).

## Patentansprüche

1. Vakuumschildanordnung, die an einem Patienten, der eine medizinische Maske trägt, angeordnet werden kann und dazu konfiguriert ist, ausgeatmete Luft vom Patienten abzuleiten, wobei die Vakuumschildanordnung (10) Folgendes umfasst:
einen Schildkörper (11), der eine konkave Konfiguration umfasst, wobei der Schildkörper (11) ferner einen Rand (12) umfasst, der ein im Wesentlichen flaches Seitenprofil des Schildkörpers (11) definiert;
ein unteres Segment (15), das an einem unteren Umfang des Schildkörpers (11) angeordnet ist, wobei das untere Segment (15) eine im Wesentlichen gekrümmte Konfiguration und eine erste Öffnung (13) umfasst;
eine Halteanordnung (20), die ausgelegt ist, um einen Sauerstoffzufuhrschlauch oder eine Vernebelungseinheit der medizinischen Maske zu halten; wobei die Halteanordnung (20) Folgendes umfasst:
eine Haltekomponente (21), die dazu konfiguriert und dimensioniert ist, einen Verbindungsteil (18) des Schildkörpers (11) zu halten, wobei der Verbindungsteil (18) in Fluidverbindung mit der ersten Öffnung (13) des Schildkörpers (11) angeordnet ist; und
einen Halterahmen (23), der eine halbzylindrische Konfiguration umfasst und mit der Haltekomponente (21) verbunden ist, wobei der Halterahmen (23) dazu konfiguriert ist, den Sauerstoffzufuhrschlauch oder eine Vernebelungseinheit der medizinischen Maske zu halten;
**dadurch gekennzeichnet, dass**:
die Halteanordnung (20) ferner ausgelegt ist, um einen Vakuumschlauch (40) zu halten;
ein unterer Abschnitt (21') der Haltekomponente (21) dazu konfiguriert und dimensioniert ist, den Vakuumschlauch (40) zu befestigen;
das untere Segment (15) dazu konfiguriert ist, einen Unterdruck auf die Innenseite des Schildkörpers (11) auszuüben; und
der Schildkörper (11) und die Halteanordnung (20) gemeinsam in eine Betriebsposition und eine Nichtbetriebsposition gebracht und aus diesen herausgenommen werden können; wobei die Betriebsposition umfasst, dass der Schildkörper (11) benachbart an die medizinische Maske und zu dieser zugewandt angeordnet ist; wobei die Betriebsposition ferner umfasst, dass der Vakuumschlauch (40) einen Unterdruck auf die Innenseite des Schildkörpers (11) ausübt, wodurch die vom Patienten ausgeatmete Luft mindestens teilweise zwischen dem Schildkörper (11) und der medizinischen Maske abgeleitet wird.

2. Vakuumschildanordnung nach Anspruch 1, wobei die Halteanordnung (20) einen ersten Verschlussmechanismus (25) und einen zweiten Verschlussmechanismus (26) umfasst, die kooperativ dazu konfiguriert sind, einen Innenbereich der Haltekomponente (21) einzustellen; wobei sowohl der erste Verschlussmechanismus (25) als auch der zweite Verschlussmechanismus (26) an dem Halterahmen (23) angeordnet sind.

3. Vakuumschildanordnung nach Anspruch 2, wobei der erste Verschlussmechanismus (25) eine Vielzahl von Verzahnungen (25') umfasst; und der zweite Verschlussmechanismus (26) einen Schnappmechanismus umfasst, der betriebsmäßig dazu konfiguriert und dimensioniert ist, um mit der Vielzahl von Verzahnungen (25') die Hülsengröße des Halterahmens (23) einzustellen.

4. Vakuumschildanordnung nach Anspruch 1, ferner umfassend mindestens ein Haltesegment (28), das an dem Halterahmen (23) angeordnet ist; wobei das mindestens eine Haltesegment (28) dazu konfiguriert ist, die Vernebelungseinheit mindestens teilweise zu halten, und wobei das mindestens eine Haltesegment (28) eine Verriegelung (29) umfasst, die an seinem oberen Ende angeordnet ist; wobei die Verriegelung (29) dazu ausgelegt ist, die Bewegung der Vernebelungseinheit in vertikaler Richtung mindestens teilweise zu reduzieren.

5. Vakuumschildanordnung nach Anspruch 1, wobei die Haltekomponente (21) einen oberen Abschnitt (21") umfasst, der dazu konfiguriert und dimensioniert ist, den Verbindungsteil (18) des Schildkörpers (11) zu halten.

6. Vakuumschildanordnung nach Anspruch 1, wobei das untere Segment (15) unterhalb des unteren Umfangs des Schildkörpers (11) angeordnet ist; wobei der Schildkörper (11) ferner ein oberes Segment umfasst, das oberhalb des unteren Segments (15) angeordnet ist; wobei das obere Segment eine Außenfläche (11'), eine Innenfläche (11") und eine zweite Öffnung (16) umfasst, die im Wesentlichen an einem mittleren Abschnitt des oberen Segments und oberhalb der ersten Öffnung (13) angeordnet ist; wobei die zweite Öffnung (16) dazu konfiguriert und dimensioniert ist, ein Verbindungssegment der medizinischen Maske aufzunehmen, wobei die medizinische Maske eine Beatmungsmaske eines Beatmungsbeutels, BVM, oder eine Beatmungsmaske mit Bedarfsventil, DVR, ist.

7. Vakuumschildanordnung nach Anspruch 6, ferner umfassend eine Vakuumbefestigung (17), die im Wesentlichen um die zweite Öffnung (16) herum angeordnet ist, wobei die Vakuumbefestigung (17) dazu konfiguriert ist, im Wesentlichen eine Dichtung zwischen der Außenfläche (11') und der Innenfläche (11") um die zweite Öffnung (16) herum zu bilden, wenn das Verbindungssegment der BVM-Beatmungsmaske oder der DVR-Maske in die zweite Öffnung (16) eingeführt wird.

## Revendications

1. Ensemble d'écran d'aspiration jetable sur un patient portant un masque médical et configuré pour éliminer l'air expiré du patient, ledit ensemble d'écran d'aspiration (10) comprenant :
un corps d'écran (11) comprenant une configuration concave, ledit corps d'écran (11) comprenant en outre un bord (12) définissant un profil latéral sensiblement plat dudit corps d'écran (11) ;
un segment inférieur (15) disposé sur un périmètre inférieur dudit corps d'écran (11), ledit segment inférieur (15) comprenant une configuration sensiblement courbe et une première ouverture (13) ;
un ensemble de retenue (20) agencé pour retenir un tube d'alimentation en oxygène ou une unité de nébuliseur du masque médical ; ledit ensemble de retenue (20) comprenant :
un composant de retenue (21) configuré et dimensionné pour retenir une portion de connexion (18) dudit corps d'écran (11), ladite portion de connexion (18) étant disposée en communication fluidique avec ladite première ouverture (13) dudit corps d'écran (11) ; et
un cadre de retenue (23) comprenant une configuration semi-cylindrique et relié audit composant de retenue (21), ledit cadre de retenue (23) étant configuré pour retenir le tube d'alimentation en oxygène ou une unité de nébuliseur du masque médical ;
**caractérisé en ce que** :
l'ensemble de retenue (20) est en outre agencé pour retenir un tube à vide (40) ;
une section inférieure (21') dudit composant de retenue (21) est configurée et dimensionnée pour la fixation du tube à vide (40) ;
ledit segment inférieur (15) est configuré pour exercer une pression négative sur un intérieur dudit corps d'écran (11) ; et
ledit corps d'écran (11) et ledit ensemble de retenue (20) sont collectivement jetables dans et hors d'une position opératoire et d'une position inopérante ; ladite position opératoire comprenant ledit corps d'écran (11) disposé de manière adjacente à et en regard du masque médical ; ladite position opératoire comprenant en outre, le tube à vide (40) exerçant une pression négative sur un intérieur dudit corps d'écran (11) en éliminant au moins partiellement l'air expiré du patient entre ledit corps d'écran (11) et le masque médical.

2. Ensemble d'écran d'aspiration selon la revendication 1, dans lequel ledit ensemble de retenue (20) comprend un premier mécanisme de fermeture (25) et un second mécanisme de fermeture (26) configurés de manière coopérative pour ajuster une zone interne du composant de retenue (21) ; chacun dudit premier mécanisme de fermeture (25) et dudit second mécanisme de fermeture (26) étant disposé sur ledit cadre de retenue (23).

3. Ensemble d'écran d'aspiration selon la revendication 2, dans lequel ledit premier mécanisme de fermeture (25) comprend une pluralité de dentelures (25') ; et ledit second mécanisme de fermeture (26) comprend un mécanisme à encliquetage configuré de manière opérative et dimensionné avec ladite pluralité de dentelures (25') pour ajuster une taille de manchon dudit cadre de retenue (23).

4. Ensemble d'écran d'aspiration selon la revendication 1, comprenant en outre au moins un segment de retenue (28) disposé sur ledit cadre de retenue (23) ; ledit au moins un segment de retenue (28) étant configuré pour retenir au moins partiellement l'unité de nébuliseur, et dans lequel ledit au moins un segment de retenue (28) comprend un loquet (29) disposé à une extrémité supérieure de celui-ci ; ledit loquet (29) étant agencé pour réduire au moins partiellement le mouvement de l'unité de nébuliseur dans une direction verticale.

5. Ensemble d'écran d'aspiration selon la revendication 1, dans lequel ledit composant de retenue (21) comprend une section supérieure (21") configurée et dimensionnée pour retenir ladite portion de connexion (18) dudit corps d'écran (11).

6. Ensemble d'écran d'aspiration selon la revendication 1, dans lequel ledit segment inférieur (15) est disposé en dessous dudit périmètre inférieur dudit corps d'écran (11) ; ledit corps d'écran (11) comprend en outre un segment supérieur disposé au-dessus dudit segment inférieur (15) ; ledit segment supérieur comprenant une surface externe (11'), une surface interne (11") et une seconde ouverture (16) disposée sensiblement sur une section médiane dudit segment supérieur et au-dessus de ladite première ouverture (13) ; ladite seconde ouverture (16) étant configurée et dimensionnée pour recevoir un segment de connexion du masque médical, le masque médical étant un masque à ballon auto-remplisseur, BVM, un masque de réanimation ou un masque de réanimation à valve à la demande, DVR.

7. Ensemble d'écran d'aspiration selon la revendication 6, comprenant en outre un raccord d'aspiration (17) disposé sensiblement autour de ladite seconde ouverture (16), dans lequel ledit raccord d'aspiration (17) est configuré pour former sensiblement une étanchéité entre ladite surface externe (11') et ladite surface interne (11") autour de ladite seconde ouverture (16) lorsque ledit segment de connexion du masque de réanimation BVM ou du masque DVR est inséré dans ladite seconde ouverture (16).
